# EUROPÄISCHE PATENTSCHRIFT

(11) **EP 4 584 603 B1**
(45) Veröffentlichungstag und Bekanntmachung des Hinweises auf die Patenterteilung: **06.05.2026**
(21) Anmeldenummer: 23761936.6
(22) Anmeldetag: 29.08.2023
(51) Int. Cl.: G01R 33/56, A61B 5/055, A61B 5/00, G06N 3/084, G06N 3/045, A61K 49/10, G01R 33/483

(54) **BESCHLEUNIGEN VON MRT-UNTERSUCHUNGEN DER LEBER**
ACCELERATION OF LIVER MRI EXAMINATIONS
ACCÉLÉRATION D'EXAMENS IRM DU FOIE

(30) Priorität: 07.09.2022 EP 22194395
(43) Veröffentlichungstag der Anmeldung: 16.07.2025
(73) Patentinhaber: Bayer Aktiengesellschaft, 51373 Leverkusen (DE); Universitätsklinikum Essen, 45147 Essen (DE)
(72) Erfinder: PIETSCH, Hubertus, 51373 Leverkusen (DE); JOST, Gregor, 51373 Leverkusen (DE); KNOBLOCH, Gesine, 51373 Leverkusen (DE); SCHÜTZ, Gunnar, 51373 Leverkusen (DE); KREIS, Felix, Karl, 51373 Leverkusen (DE); LIENERTH, Christian, 51373 Leverkusen (DE); HAUBOLD, Johannes, 45147 Essen (DE); HOSCH, René, 45147 Essen (DE); NENSA, Felix, 45147 Essen (DE); FORSTING, Michael, 45147 Essen (DE)
(74) Vertreter: BIP Patents
(86) Internationale Anmeldenummer: PCT/EP2023/073597
(87) Internationale Veröffentlichungsnummer: WO 2024/052157

(56) Entgegenhaltungen:
- WO-A1-2021/052896

## Beschreibung

Die vorliegende Erfindung befasst sich mit der Beschleunigung einer MRT-Untersuchung der Leber mit Hilfe eines Modells des maschinellen Lernens. Das Modell des maschinellen Lernens ist konfiguriert und trainiert, eine MRT-Aufnahme einer Leber in der hepatobiliären Phase nach der Applikation eines hepatobiliären Kontrastmittels auf Basis einer oder mehrerer MRT-Aufnahmen, die zu einem Zeitpunkt in einer früheren Phase erzeugt worden sind, vorherzusagen. Gegenstände der vorliegenden Erfindung sind ein computer-implementiertes Verfahren zum Vorhersagen der MRT-Aufnahme in der hepatobiliären Phase mittels des trainierten Modells sowie ein Computersystem und ein Computerprogrammprodukt zum Ausführen des Vorhersageverfahrens.

Die zeitliche Verfolgung von Vorgängen innerhalb des Körpers eines Menschen oder eines Tieres mit bildgebenden Verfahren spielt unter anderem bei der Diagnose und/oder Therapie von Krankheiten eine wichtige Rolle.

Als Beispiel sei die Detektion und Differentialdiagnose fokaler Leberläsionen mittels dynamischer kontrastverstärkender Magnetresonanztomographie (MRT) mit einem hepatobiliären Kontrastmittel genannt.

Ein hepatobiliäres Kontrastmittel wie beispielsweise Primovist^{®} kann zur Detektion von Tumoren in der Leber eingesetzt werden. Die Blutversorgung des gesunden Lebergewebes erfolgt in erster Linie über die Pfortader (*Vena portae*), während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist^{®} kann zur Detektion und Differenzierung gutartiger und bösartiger fokaler Leberläsionen eingesetzt werden. Es liefert mittels T1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Bei der durch Primovist^{®} erzielten Kontrastverstärkung während der Anflutungsphase beobachtet man typische Anreicherungsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten MRT-Aufnahmen führt Primovist^{®} 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als T1-hypointensere Bereiche auftreten, während bspw. fokal noduläre Hyperplasien Kontrastmittel in dieser Phase anreichern.

Die zeitliche Verfolgung der Verteilung des Kontrastmittels bietet also eine gute Möglichkeit der Detektion und Differentialdiagnose fokaler Leberläsionen, allerdings zieht sich die Untersuchung über eine vergleichsweise lange Zeitspanne hin. Über diese Zeitspanne sollten Bewegungen des Patienten weitgehend vermieden werden, um Bewegungsartefakte in den MRT-Aufnahmen zu minimieren. Die lang andauernde Bewegungseinschränkung kann für einen Patienten unangenehm sein.

In der Offenlegungsschrift WO2021/052896A1 wird vorgeschlagen, eine oder mehrere MRT-Aufnahmen während der hepatobiliären Phase nicht messtechnisch zu erzeugen, sondern auf Basis von MRT-Aufnahmen aus einer Mehrzahl an vorangegangenen Phasen zu berechnen (vorherzusagen), um die Aufenthaltsdauer des Patienten im MRT-Scanner zu verkürzen.

Überraschend wurde gefunden, dass zur Vorhersage einer MRT-Aufnahme der Leber in der hepatobiliären Phase keine MRT-Aufnahmen aus einer Mehrzahl an unterschiedlichen Phasen erforderlich ist. Überraschend wurde gefunden, dass die Qualität der vorhergesagten MRT-Aufnahme gleichwertig ist, wenn die Vorhersage lediglich auf Basis einer oder mehrerer kontrastmittelverstärkten MRT-Aufnahmen, die die Leber zu einem Zeitpunkt in der Übergangsphase repräsentiert/repräsentieren, und optional einer oder mehrerer nativen MRT-Aufnahmen erfolgt.

Ein erster Gegenstand der vorliegenden Erfindung ist daher ein computer-implementiertes Verfahren zur Vorhersage einer MRT-Aufnahme gemäß Anspruch 1, wobei das Verfahren unter anderem umfasst:
- Empfangen von Patientendaten, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme die Leber oder den Teil der Leber des Patienten ohne Kontrastmittel repräsentiert,
- Eingeben der Patientendaten in ein trainiertes Modell des maschinellen Lernens, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel,
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- Empfangen einer vorhergesagten MRT-Aufnahme von dem trainierten Modell des maschinellen Lernens, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- Ausgeben und/oder Speichern der vorhergesagten MRT-Aufnahme und/oder Übermitteln der vorhergesagten MRT-Aufnahme an ein separates Computersystem.

Ein weiterer Gegenstand der vorliegenden Erfindung ist ein Computersystem umfassend
- eine Eingabeeinheit,
- eine Steuer- und Recheneinheit und
- eine Ausgabeeinheit,
wobei die Steuer- und Recheneinheit konfiguriert ist,
- die Eingabeeinheit zu veranlassen, Patientendaten zu empfangen, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme die Leber oder den Teil der Leber des Patienten ohne Kontrastmittel repräsentiert,
- die Patientendaten in ein trainiertes Modell des maschinellen Lernens einzugeben, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- eine vorhergesagte MRT-Aufnahme von dem trainierten Modell des maschinellen Lernens zu empfangen, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- die Ausgabeeinheit zu veranlassen, die vorhergesagte MRT-Aufnahme auszugeben und/oder zu speichern und/oder an ein separates Computersystem zu übermitteln.

Ein weiterer Gegenstand der vorliegenden Erfindung ist ein Computerprogrammprodukt umfassend einen Datenspeicher, in dem ein Computerprogramm gespeichert ist, das in einen Arbeitsspeicher eines Computersystems geladen werden kann und dort das Computersystem dazu veranlasst, folgende Schritte ausführen:
- Empfangen von Patientendaten, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme die Leber oder den Teil der Leber des Patienten ohne Kontrastmittel repräsentiert,
- Eingeben der Patientendaten in ein trainiertes Modell des maschinellen Lernens, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- Empfangen einer vorhergesagten MRT-Aufnahme von dem trainierten Modell des maschinellen Lernens, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- Ausgeben und/oder Speichern der vorhergesagten MRT-Aufnahme und/oder Übermitteln der vorhergesagten MRT-Aufnahme an ein separates Computersystem.

Ein weiterer Gegenstand der vorliegenden Erfindung ist eine Verwendung eines hepatobiliären Kontrastmittels in einem MRT-Untersuchungsverfahren umfassend
- Empfangen von Patientendaten, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation des hepatobiliären Kontrastmittels repräsentiert, wobei die optionale mindestens eine native MRT-Aufnahme die Leber oder den Teil der Leber des Patienten ohne Kontrastmittel repräsentiert,
- Eingeben der Patientendaten in ein trainiertes Modell des maschinellen Lernens, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel,
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- Empfangen einer vorhergesagten MRT-Aufnahme von dem trainierten Modell des maschinellen Lernens, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- Ausgeben und/oder Speichern der vorhergesagten MRT-Aufnahme und/oder Übermitteln der vorhergesagten MRT-Aufnahme an ein separates Computersystem.

Ein weiterer Gegenstand der vorliegenden Erfindung ist ein Kit umfassend ein hepatobiliäres Kontrastmittel und ein Computerprogrammprodukt, wobei das Computerprogrammprodukt einen Datenspeicher umfasst, in dem ein Computerprogramm gespeichert ist, das in einen Arbeitsspeicher eines Computersystems geladen werden kann und dort das Computersystem dazu veranlasst, folgende Schritte ausführen:
- Empfangen von Patientendaten, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation des hepatobiliären Kontrastmittels repräsentiert, wobei die optionale mindestens eine native MRT-Aufnahme die Leber oder den Teil der Leber des Patienten ohne Kontrastmittel repräsentiert,
- Eingeben der Patientendaten in ein trainiertes Modell des maschinellen Lernens, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel,
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- Empfangen einer vorhergesagten MRT-Aufnahme von dem trainierten Modell des maschinellen Lernens, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- Ausgeben und/oder Speichern der vorhergesagten MRT-Aufnahme und/oder Übermitteln der vorhergesagten MRT-Aufnahme an ein separates Computersystem.

Die Erfindung wird nachstehend näher erläutert, ohne zwischen den Erfindungsgegenständen (Vorhersageverfahren, Computersystem, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Vorhersageverfahren, Computersystem, Computerprogrammprodukt, Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, sofern der daraus resultierende Gegenstand in den Schutzbereich der beigefügten Ansprüche fällt, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die lediglich durch die Ansprüche definierte Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Mit Hilfe der vorliegenden Erfindung kann eine MRT-Aufnahme eines Untersuchungsbereichs eines Untersuchungsobjekts vorhergesagt werden. In dieser Beschreibung wird die Vorhersage einer MRT-Aufnahme synonym auch als Erzeugen einer vorhergesagten MRT-Aufnahme bezeichnet.

Das "Untersuchungsobjekt" ist üblicherweise ein Lebewesen, vorzugsweise ein Säugetier, ganz besonders bevorzugt ein Mensch. Das Untersuchungsobjekt wird in dieser Beschreibung auch als Patient bezeichnet
Der "Untersuchungsbereich" ist eine Leber oder ein Teil der Leber des Untersuchungsobjekts.

In einer bevorzugten Ausführungsform der vorliegenden Erfindung ist der Untersuchungsbereich die Leber oder ein Teil der Leber eines Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Die Magnetresonanztomographie, abgekürzt MRT oder MRI (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird

Bei der MRT-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MRT-Daten liegen zunächst als Rohdaten im Frequenzraum vor, und können durch anschließende inverse Fourier-Transformation in den Ortsraum (Bildraum) transformiert werden.

Die vorhergesagte MRT-Aufnahme kann eine Repräsentation des Untersuchungsbereichs im Ortsraum (Bildraum) oder eine Repräsentation im Frequenzraum oder eine andere Repräsentation sein. Vorzugsweise liegen MRT-Aufnahmen, die zum Trainieren des Modells des maschinellen Lernens und zur Vorhersage verwendet werden, in einer Ortsraum-Darstellung oder in einer Form vor, die sich in eine Ortsraum-Darstellung umwandeln (transformieren) lässt.

Eine MRT-Aufnahme im Sinne der vorliegenden Erfindung kann eine zweidimensionale, dreidimensionale oder höherdimensionale Repräsentation sein. Üblicherweise liegen zweidimensionale (2D) Tomogramme (Schichtaufnahmen) vor oder es liegt ein Stapel von zweidimensionalen Tomogrammen (Schichtaufnahmen) vor oder es liegt eine dreidimensionale (3D) Repräsentation vor. Üblicherweise liegen die MRT-Aufnahmen in digitaler Form vor. Der Begriff "digital" bedeutet, dass die Repräsentationen von einer Maschine, in der Regel einem Computersystem, verarbeitet werden können. Unter "Verarbeitung" werden die bekannten Verfahren zur elektronischen Datenverarbeitung (EDV) verstanden. Ein Beispiel für ein übliches Format für eine digitale MRT-Aufnahme ist das DICOM-Format (DICOM: *Digital Imaging and Communications in Medicine*) - ein offener Standard zur Speicherung und zum Austausch von Informationen im medizinischen Bilddatenmanagement.

Aus Gründen der einfacheren Darstellung wird die Erfindung an einigen Stellen der vorliegenden Beschreibung anhand des Vorliegens von zweidimensionalen Bildern erläutert, ohne die Erfindung jedoch auf zweidimensionale Bilder beschränken zu wollen. Dem Fachmann ist klar, wie sich das jeweils Beschriebene auf Stapel zweidimensionaler Bilder, auf 3D-Aufnahmen oder auf Repräsentationen im Frequenzraum übertragen lässt.

Die vorhergesagte MRT-Aufnahme repräsentiert die Leber oder den Teil der Leber des Untersuchungsobjekts in der hepatobiliären Phase nach der Applikation eines hepatobiliären Kontrastmittels.

Unter einem hepatobiliären Kontrastmittel wird ein Kontrastmittel verstanden, das spezifisch von gesunden Leberzellen, den Hepatozyten, aufgenommen wird.

Bespiele für hepatobiliäre Kontrastmittel sind Kontrastmittel auf der Basis der Gadoxetsäure. Sie sind beispielsweise in US 6,039,931A beschrieben. Sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

Ein weiteres MRT-Kontrastmittel mit einer geringeren Aufnahme in die Hepatozyten ist Gadobenate Dimeglumine (Multihance^{®}).

Weitere hepatobiliäre Kontrastmittel sind u.a. in WO2022/194777 beschrieben.

In einer Ausführungsform der vorliegenden Offenbarung handelt es sich bei dem verwendeten Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl)triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

Nach der intravenösen Applikation des hepatobiliären Kontrastmittels in Form eines Bolus in eine Armvene erreicht das Kontrastmittel die Leber zunächst über die Arterien. Diese sind in T1-gewichteten MRT-Aufnahmen signalverstärkt dargestellt. Die Phase, in der die Leberarterien in T1-gewichteten MRT-Aufnahmen signalverstärkt dargestellt sind, wird als "arterielle Phase" bezeichnet.

Anschließend erreicht das Kontrastmittel die Leber über die Lebervenen. Während die Signalverstärkung in den Leberarterien bereits abnimmt, erreicht die Signalverstärkung in den Lebervenen ein Maximum. Die Phase, in der die Lebervenen in T1-gewichteten MRT-Aufnahmen signalverstärkt dargestellt sind, wird als "portalvenöse Phase" bezeichnet. Diese Phase kann bereits während der arteriellen Phase beginnen und sich mit dieser überlagern.

An die portalvenöse Phase schließt sich die "Übergangsphase" (engl. *transitional phase*) an, in der die Signalverstärkung in den Leberarterien weiter absinkt, die Signalverstärkung in den Lebervenen ebenfalls absinkt. Bei Verwendung eines hepatobiliären Kontrastmittels steigt die Signalverstärkung in den gesunden Leberzellen in der Übergangsphase allmählich an.

Die arterielle Phase, die portalvenöse Phase und die Übergangsphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

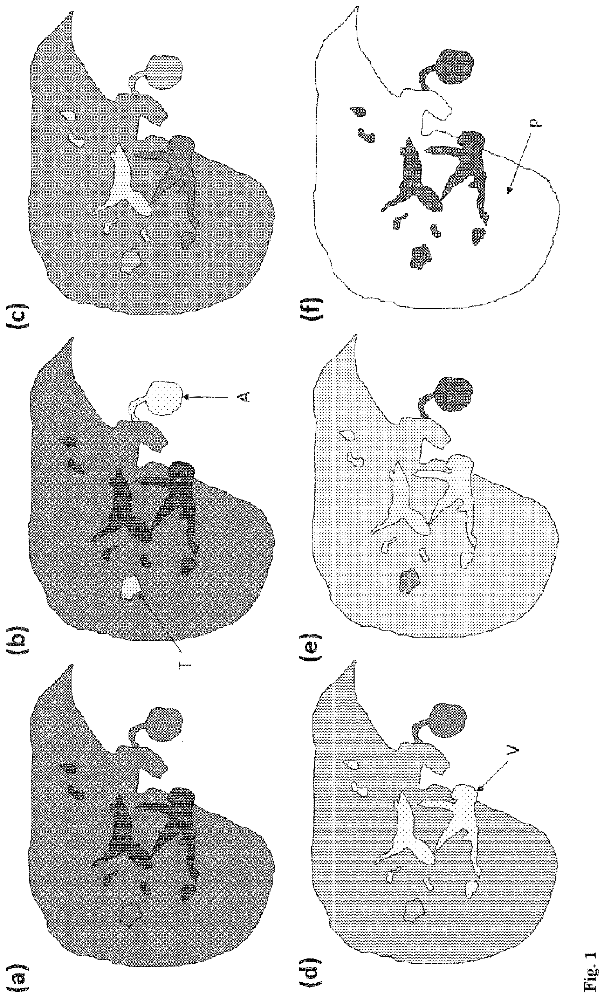

Fig. 1 zeigt schematisch und beispielhaft eine Mehrzahl an T1-gewichteten MRT-Aufnahmen eines Teils der Leber eines Untersuchungsobjekts während der dynamischen Phase und der hepatobiliären Phase. In den Figuren 1 (a), 1 (b), 1 (c), 1 (d), 1 (e) und 1 (f) ist stets derselbe Querschnitt durch die Leber zu unterschiedlichen Zeitpunkten dargestellt. Die in den Figuren 1 (b), 1 (d) und 1 (f) eingezeichneten Bezugszeichen gelten für alle Figuren 1 (a), 1 (b), 1 (c), 1 (d), 1 (e) und 1 (f); sie sind lediglich der besseren Übersicht halber jeweils nur einmal eingezeichnet.

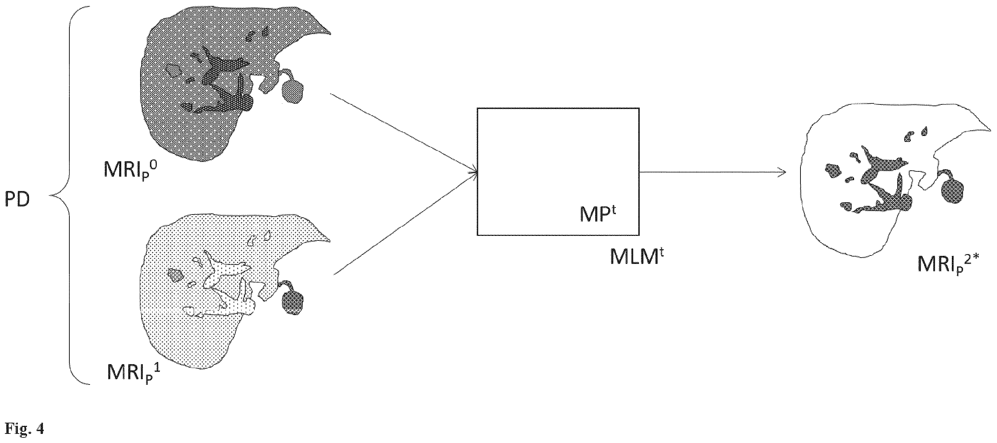

Fig. 1 (a) zeigt den Querschnitt durch die Leber vor der intravenösen Applikation eines hepatobiliären Kontrastmittels (native MRT-Aufnahme). Zu einem Zeitpunkt, der zwischen den Zeitpunkten liegt, die durch die Figuren 1 (a) und 1 (b) dargestellt werden, wurde ein hepatobiliäres Kontrastmittel intravenös als Bolus verabreicht. Dieses erreicht in Fig. 1 (b) über die Leberarterie (A) die Leber. Dementsprechend wird die Leberarterie signalverstärkt dargestellt (arterielle Phase). Ein Tumor (T), der hauptsächlich über Arterien mit Blut versorgt wird, hebt sich ebenfalls als hellerer (signalverstärkter) Bereich vor dem Leberzellengewebe ab. Zu dem Zeitpunkt, der in Figur 1 (c) dargestellt ist, erreicht das Kontrastmittel die Leber über die Venen. In Figur 1 (d) heben sich die venösen Blutgefäße (V) als helle (signalverstärkte) Bereiche von dem Lebergewebe ab (venöse Phase). Gleichzeitig steigt die Signalintensität in den gesunden Leberzellen, die hauptsächlich über die Venen mit Kontrastmittel versorgt werden, kontinuierlich an (Fig. 1 (c) → 1 (d) → 1 (e) → 1 (f)). Fig. 1 (e) zeigt eine T1-gewichtete MRT-Aufnahme in der Übergangsphase. Es ist bereits deutlich eine Signalanhebung in den gesunden Leberzellen (P) zu erkennen, während das Signal in den Venen bereits wieder abnimmt. In der hepatobiliären Phase, die in Fig. 4 (f) dargestellt ist, sind die gesunden Leberzellen (P) signalverstärkt dargestellt; die Blutgefäße und der Tumor weisen kein Kontrastmittel mehr auf und sind entsprechend dunkel dargestellt.

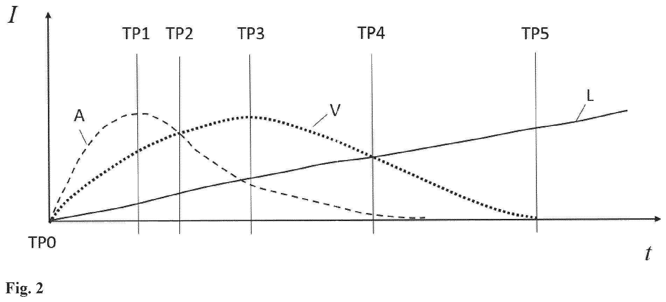

Fig. 2 zeigt schematisch den zeitlichen Verlauf (*t* = Zeit) der Signalintensitäten *I,* die durch ein hepatobiliäres Kontrastmittel in Leberarterien (A), Lebervenen (V) und gesunden Leberzellen (P) bei einer kontrastverstärkten MRT-Untersuchung hervorgerufen werden. Die Signalintensität *I* korreliert positiv mit der Konzentration des Kontrastmittels in den genannten Bereichen. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (V) steigt langsamer an als in den Leberarterien und erreicht ihr Maximum später (gepunktete Kurve). Die Konzentration des Kontrastmittels in den gesunden Leberzellen (P) steigt langsam an (durchgezogene Kurve) und erreicht ihr Maximum erst zu einem sehr viel späteren Zeitpunkt (in der Figur 1 nicht dargestellt). Es lassen sich einige charakteristische Zeitpunkte definieren: Zum Zeitpunkt TP0 wird Kontrastmittel intravenös als Bolus appliziert. Da die Applikation eines Kontrastmittels selbst eine gewisse Zeitspanne in Anspruch nimmt, definiert der Zeitpunkt TP0 vorzugsweise den Zeitpunkt, zu dem die Applikation abgeschlossen ist, Kontrastmittel also vollständig in das Untersuchungsobjekt eingebracht ist. Zum Zeitpunkt TP1 erreicht die Signalintensität des Kontrastmittels in den Leberarterien (A) ihr Maximum. Zum Zeitpunkt TP2 schneiden sich die Kurven der Signalintensitäten bei den Leberarterien (A) und den Lebervenen (V). Zum Zeitpunkt TP3 durchläuft die Signalintensität des Kontrastmittels in den Lebervenen (V) ihr Maximum. Zum Zeitpunkt TP4 schneiden sich die Kurven der Signalintensitäten bei den Leberarterien (A) und den gesunden Leberzellen (P). Zum Zeitpunkt TP5 sind die Konzentrationen in den Leberarterien (A) und den Lebervenen (V) auf ein Niveau gesunken, bei dem sie keine messbare Kontrastverstärkung mehr verursachen.

Die Vorhersage einer MRT-Aufnahme in der hepatobiliären Phase erfolgt auf Basis mindestens einer MRT-Aufnahme, wobei die mindestens eine MRT-Aufnahme auf i) mindestens eine erste MRT-Aufnahme in der Übergangsphase und ii) optional mindestens eine native MRT-Aufnahme beschränkt ist. Mit anderen Worten: für die Vorhersage werden keine MRT-Aufnahmen aus der arteriellen Phase und/oder der portalvenösen Phase herangezogen. Die mindestens eine native MRT-Aufnahme zeigt die Leber oder den Teil der Leber ohne Kontrastmittel. Sie wird vorzugsweise vor der Applikation des hepatobiliären Kontrastmittels erzeugt.

Wie in der internationalen Patentanmeldung WO2022223383A1 (PCT/EP2022/059836) beschrieben können Bildregistrierungsfehler vermindert werden, wenn nicht nur eine native MRT-Aufnahme zur Vorhersage verwendet wird, sondern mehr als eine native MRT-Aufnahme verwendet wird (z.B. zwei oder drei native MRT-Aufnahmen). Bei der Verwendung von mehr als einer nativen MRT-Aufnahme lernt das Modell des maschinellen Lernens beim Training, Bewegungsartefakte auszugleichen.

Die Vorhersage der MRT-Aufnahme in der hepatobiliären Phase erfolgt mit Hilfe eines Modells des maschinellen Lernens.

Ein "Modell des maschinellen Lernens" kann als eine computerimplementierte Datenverarbeitungsarchitektur verstanden werden. Das Modell kann Eingabedaten empfangen und Ausgabedaten auf der Grundlage dieser Eingabedaten und Modellparametern liefern. Das Modell kann durch Training eine Beziehung zwischen den Eingabedaten und den Ausgabedaten erlernen. Beim Training können die Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden.

Im Training kann eine Fehlerfunktion (engl.: *loss function*) verwendet werden, um den Trainingsprozess zu steuern und die Vorhersagequalität des Modells zu bewerten. Die Fehlerfunktion kann so gewählt werden, dass sie eine erwünschte Beziehung zwischen Ausgabedaten und Zieldaten "belohnt" und/oder eine unerwünschte Beziehung zwischen Ausgabedaten und Zieldaten "bestraft". Eine solche Beziehung kann z.B. eine Ähnlichkeit, eine Unähnlichkeit oder eine andere Beziehung sein.

Eine Fehlerfunktion kann verwendet werden, um Fehler (engl.: *loss*) für ein gegebenes Paar aus Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Eine Fehlerfunktion kann z.B. die Abweichung zwischen den Ausgabedaten des Modells für bestimmte Eingabedaten und den Zieldaten quantifizieren. Handelt es sich bei den Ausgabedaten und den Zieldaten beispielsweise um Zahlen, kann die Fehlerfunktion die absolute Differenz zwischen diesen Zahlen sein.

In diesem Fall kann ein hoher absoluter Wert der Fehlerfunktion bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall umfassen die Trainingsdaten für jedes Untersuchungsobjekt einer Vielzahl an Untersuchungsobjekten MRT-Aufnahmen, wobei die MRT-Aufnahmen beschränkt sind auf:
∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
∘ optional mindestens eine native MRT-Aufnahme der Leber oder des Teils der Leber des Untersuchungsobjekts ohne Kontrastmittel und
∘ eine zweite MRT-Aufnahme, wobei die zweite MRT-Aufnahme die Leber oder den Teil der Leber des Untersuchungsobjekts in der hepatobiliären Phase nach der Applikation des hepatobiliären Kontrastmittels repräsentiert.

Der Begriff "Vielzahl an Untersuchungsobjekten" bedeutet mindestens zehn vorzugsweise mindestens einhundert Untersuchungsobjekte.

Die mindestens eine erste MRT-Aufnahme und die optionale mindestens eine native MRT-Aufnahme dienen als Eingabedaten für das Modell des maschinellen Lernens. Die zweite MRT-Aufnahme dient als Zieldaten.

Vorzugsweise handelt es sich bei der mindestens einen ersten MRT-Aufnahme um mindestens eine Repräsentation des Untersuchungsbereichs des Untersuchungsobjekts zu einem Zeitpunkt im Bereich von 3 Minuten bis 6 Minuten nach der Applikation des hepatobiliären Kontrastmittels. Besonders bevorzugt handelt es sich bei der mindestens einen ersten MRT-Aufnahme um mindestens eine Repräsentation des Untersuchungsbereichs des Untersuchungsobjekts zu einem Zeitpunkt im Bereich von 3 Minuten bis 5 Minuten nach der Applikation des hepatobiliären Kontrastmittels. Besonders bevorzugt handelt es sich bei der mindestens einen ersten MRT-Aufnahme um mindestens eine Repräsentation des Untersuchungsbereichs des Untersuchungsobjekts zu einem Zeitpunkt im Bereich zwischen den in Fig. 2 gezeigten Zeitpunkten TP3 und TP5.

Unter "Applikation" wird in dieser Beschreibung vorzugsweise die intravenöse Applikation des Kontrastmittels als Bolus in eine Armvene des Patienten verstanden.

Gadoxetat-Dinatrium (GD, Primovist^{®}) ist beispielsweise in einer Dosis von 0,1 ml / kg Körpergewicht (BW) (0,025 mmol / kg BW Gd) zugelassen. Die empfohlene Verabreichung (Applikation) von GD umfasst eine unverdünnte intravenöse Bolusinjektion mit einer Flussrate von ungefähr 2 ml / Sekunde, gefolgt von einer Spülung der i.v. Kanüle mit einer physiologischen Kochsalzlösung.

Vorzugsweise repräsentiert die mindestens eine erste MRT-Aufnahme den Untersuchungsbereich zu einem definierten Zeitpunkt bzw. zu einer definierten Zeitspanne, wobei die Zeitspanne der Dauer der Aufnahme von Rohdaten zur Erzeugung der mindestens einen ersten MRT-Aufnahme entspricht.

Vorzugsweise handelt es sich bei der mindestens einen ersten MRT-Aufnahme um eine MRT-Aufnahme, die zu einem Zeitpunkt nach dem Zeitpunkt TP3 (siehe Figur 2) erzeugt wurde.

Vorzugsweise handelt es sich bei der mindestens einen ersten MRT-Aufnahme um eine MRT-Aufnahme, die zu einem Zeitpunkt nach dem Zeitpunkt TP3 und vor dem Zeitpunkt TP5 (siehe Figur 2) erzeugt wurde.

Vorzugsweise handelt es sich bei der mindestens einen ersten MRT-Aufnahme um eine MRT-Aufnahme, die zu einem Zeitpunkt zwischen 30 Sekunden vor dem Zeitpunkt TP4 und 30 Sekunden nach dem Zeitpunkt TP4 (siehe Figur 2) erzeugt wurde.

Vorzugsweise umfasst die mindestens eine erste MRT-Aufnahme eine T1-gewichtete MRT-Aufnahme des Untersuchungsbereichs.

Vorzugsweise ist die mindestens eine erste MRT-Aufnahme das Ergebnis einer Dixon-Sequenz, vorzugweise einer T1-gewichteten Dixon-Sequenz, vorzugsweise mit In- und Opposed-phase (Wasser zu Fett) Aufnahmetechnik (siehe z.B. A. S. Shetty: In-Phase and Opposed-Phase Imaging: Applications of Chemical Shift and Magnetic Susceptibility in the Chest and Abdomen, RadioGraphics 2019, 39:115-135) und/oder die mindestens eine erste MRT-Aufnahme umfasst ein solches Ergebnis.

Vorzugsweise umfasst die mindestens eine erste MRT-Aufnahme eine In-Phase-Aufnahme und eine Opposed-Phase-Aufnahme sowie eine Fettkarte (engl.: *fat-only image*) und eine Wasserkarte (engl.: *water-only image*).

Vorzugsweise umfasst die mindestens eine native MRT-Aufnahme eine T1-gewichtete MRT-Aufnahme des Untersuchungsbereichs.

Vorzugsweise ist die mindestens eine native MRT-Aufnahme das Ergebnis einer Dixon-Sequenz, vorzugweise einer T1-gewichteten Dixon-Sequenz, vorzugsweise mit In- und Opposed-Phase (Wasser zu Fett) Aufnahmetechnik.

Vorzugsweise ist die mindestens eine native MRT-Aufnahme eine Wasserkarte (engl.: *water-only image*) des Untersuchungsbereichs oder umfasst eine solche.

Vorzugsweise ist die zweite MRT-Aufnahme eine Repräsentation der Leber zu einem Zeitpunkt im Bereich von 10 Minuten bis 30 Minuten nach der Applikation des hepatobiliären Kontrastmittels. Besonders bevorzugt handelt es sich bei der zweiten MRT-Aufnahme um eine Repräsentation des Untersuchungsbereichs des Untersuchungsobjekts zu einem Zeitpunkt im Bereich von 15 Minuten bis 20 Minuten nach der Applikation des hepatobiliären Kontrastmittels.

Vorzugsweise repräsentiert die zweite MRT-Aufnahme den Untersuchungsbereich zu einem definierten Zeitpunkt bzw. in einer definierten Zeitspanne in der hepatobiliären Phase, wobei die Zeitspanne der Dauer der Aufnahme von Rohdaten zur Erzeugung der zweiten MRT-Aufnahme entspricht.

Vorzugsweise ist die zweite MRT-Aufnahme eine T1-gewichtete MRT-Aufnahme. Vorzugsweise ist die zweite MRT-Aufnahme eine Wasserkarte (engl.: *water-only image*) des Untersuchungsbereichs oder umfasst eine solche.

Vorzugsweise wurden für die mindestens eine erste MRT-Aufnahme, die optionale mindestens eine native MRT-Aufnahme und die zweite MRT-Aufnahme die gleichen Sequenzen zu ihrer Aufnahme verwendet und identische konstrastbestimmende Messparameter (z.B. Repetionsszeit, Echozeiten, Flipwinkel, Methode der Fettunterdrückung) verwendet, damit die Unterschiede im Bildkontrast nur vom Kontrastmittel herrühren.

Bevor MRT-Aufnahmen dem Modell des maschinellen Lernens zugeführt werden, erfolgt üblicherweise eine Registrierung.

Eine solche Registrierung (auch "Co-Registrierung" oder Bildregistrierung genannt) ist ein Prozess in der digitalen Bildverarbeitung und dient dazu, zwei oder mehrere Aufnahmen (z.B. Bilder) derselben Szene, oder zumindest ähnlicher Szenen, bestmöglich miteinander in Übereinstimmung zu bringen. Dabei wird eine der Aufnahmen als Referenzaufnahme festgelegt, die anderen werden Objektaufnahmen genannt. Um diese Objektaufnahmen optimal an die Referenzaufnahme anzupassen, wird eine ausgleichende Transformation berechnet. Die zu registrierenden Aufnahmen unterscheiden sich voneinander, weil sie von unterschiedlichen Positionen, zu unterschiedlichen Zeitpunkten oder mit unterschiedlichen Sensoren aufgenommen wurden.

Im Fall der MRT-Aufnahmen der vorliegenden Erfindung wurden sie zu unterschiedlichen Zeitpunkten aufgenommen.

Das Ziel der Bildregistrierung ist also, diejenige Transformation zu finden, die eine gegebene Objektaufnahme bestmöglich mit der Referenzaufnahme in Übereinstimmung bringt. Das Ziel ist, dass nach Möglichkeit jedes Pixel/Voxel einer Aufnahme denselben Untersuchungsbereich in einem Untersuchungsobjekt repräsentiert wie das Pixel/Voxel einer anderen (co-registrierten) Aufnahme mit denselben Koordinaten.

Verfahren zur Bildregistrierung sind im Stand der Technik beschrieben (siehe z.B.: E.H. Seeley et al.: Co-registration of multi-modality imaging allows for comprehensive analysis of tumor-induced bone disease, Bone 2014, 61, 208-216; C. Bhushan et al.: Co-registration and distortion correction of diffusion and anatomical images based on inverse contrast normalization, Neuroimage 2015, 15, 115: 269-80; US20200214619; US20090135191; EP3639272A).

Es ist möglich, dass die MRT-Aufnahmen weiteren Bildbearbeitungsverfahren unterworfen werden, wie z.B. Filterungen, Upsampling, Downsampling, Reduktion auf einen Bereich von Interesse und/oder andere.

Es ist denkbar, dass die Vorhersage nicht ausschließlich auf der mindestens einen ersten MRT-Aufnahme und optional auf der mindestens einen nativen MRT-Aufnahme basiert, sondern dass weitere Daten als weitere Eingabedaten verwendet werden. Solche weiteren Daten können Informationen zum Untersuchungsobjekt umfassen (z.B. Alter, Geschlecht, Körpergröße, Körpergewicht, Body-Mass-Index, Ruheherzfrequenz, Herzfrequenzvariabilität, Körpertemperatur, Informationen über den Lebensstil des Untersuchungsobjekts, wie z.B. Alkoholkonsum, Rauchen und/oder körperliche Betätigung und/oder die Ernährung des Untersuchungsobjekts, medizinische Interventionsparameter wie regelmäßige Medikation, gelegentliche Medikation oder andere frühere oder aktuelle medizinische Interventionen und/oder andere Informationen über frühere und aktuelle Behandlungen des Untersuchungsobjekts und berichtete Gesundheitszustände und/oder Kombinationen davon), Informationen über den Untersuchungsbereich (z.B. Größe und/oder Form des Untersuchungsbereichs und/oder die Lage des Untersuchungsbereichs innerhalb des Untersuchungsobjekts) und/oder Informationen über die mindestens eine MRT-Aufnahme (z.B. unter welchen Bedingungen sie erzeugt worden ist und/oder welche Messparameter und/oder Messsequenzen verwendet wurden, um sie zu erzeugen).

Während des Trainings des Modells des maschinellen Lernens werden für jedes Untersuchungsobjekt der Vielzahl an Untersuchungsobjekten die mindestens eine erste MRT-Aufnahme und optional die mindestens eine native MRT-Aufnahme und optional weitere Daten als Eingabedaten in das Modell des maschinellen Lernens eingegeben. Das Modell des maschinellen Lernens ist konfiguriert, auf Basis der Eingabedaten und auf Basis von Modellparametern, Ausgabedaten zu erzeugen. Die Ausgabedaten stellen eine vorhergesagte zweite MRT-Aufnahme dar, das heißt eine vorhergesagte MRT-Aufnahme des Untersuchungsbereichs des Untersuchungsobjekt in der hepatobiliären Phase.

Die vorhergesagte zweite MRT-Aufnahme wird für jedes Untersuchungsobjekt mit der jeweiligen (vorzugsweise gemessenen) zweiten MRT-Aufnahme verglichen. Mit Hilfe einer Fehlerfunktion können die Abweichungen quantifiziert werden. Geeignete Fehlerfunktionen zur Quantifizierung von Abweichungen zwischen zwei MRT-Aufnahmen sind zum Beispiel L1-Fehlerfunktion (*L1 loss*), L2-Fehlerfunktion (*L2 loss*), Lp-Fehlerfunktion (*Lp loss*), Strukturähnlichkeitsindexmaß (*structural similarity index measure* (SSIM)), VGG-Fehlerfunktion (*VGG loss*), Wahrnehmungs-Fehlerfunktion (*perceptual loss*) oder eine Kombination der oben genannten Funktionen oder andere Fehlerfunktionen. Weitere Einzelheiten zu Fehlerfunktionen sind z.B. in der wissenschaftlichen Literatur zu finden (siehe z. B.: R. Mechrez et al.: The Contextual Loss for Image Transformation with Non-Aligned Data, 2018, arXiv:1803.02077v4; H. Zhao et al.: Loss Functions for Image Restoration with Neural Networks, 2018, arXiv:1511.08861v3).

Mit Hilfe eines Gradientenverfahrens oder eines anderen Optimierungsverfahrens können die Modellparameter so modifiziert werden, dass der Fehler für alle Paare reduziert und vorzugsweise unter einen vordefinierten Schwellenwert fällt und/oder auf ein definiertes Minimum reduziert wird.

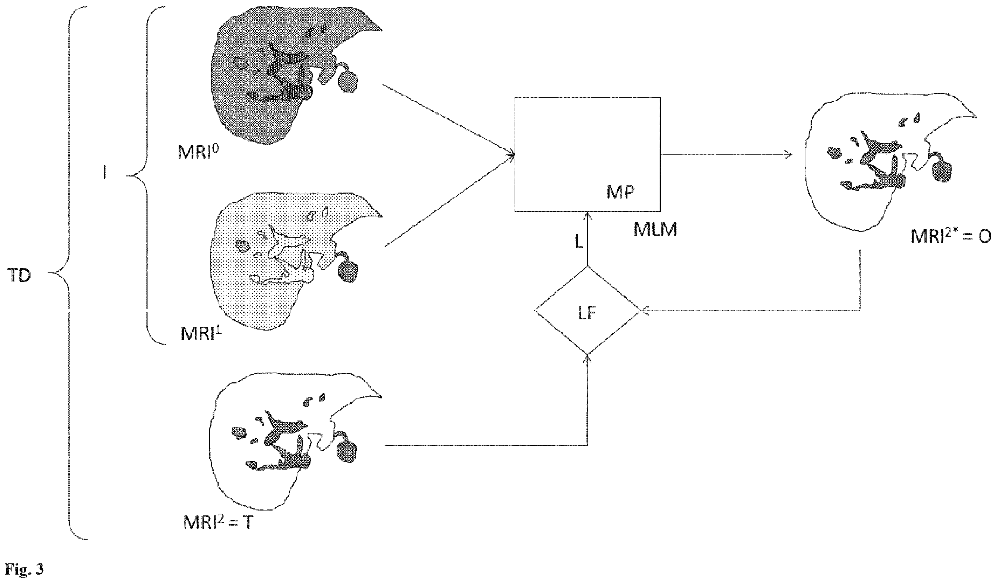

Fig. 3 zeigt beispielhaft und schematisch eine Ausführungsform des Verfahrens zum Trainieren des Modells des maschinellen Lernens.

Das Modell des maschinellen Lernens (MLM) wird mit Hilfe von Trainingsdaten (TD) trainiert. Üblicherweise umfassen die Trainingsdaten für jedes Untersuchungsobjekt einer Vielzahl von Untersuchungsobjekten Eingabedaten und Zieldaten. In Fig. 3 ist nur ein Datensatz eines Untersuchungsobjekts gezeigt, der aus den Eingabedaten (I) und den Zieldaten (T) besteht. Die Eingabedaten (I) bestehen im vorliegenden Beispiel aus mindestens einer ersten MRT-Aufnahme (MRI¹), die die Leber des Untersuchungsobjekts zu einem Zeitpunkt in der Übergangsphase nach der Applikation eines hepatobiliären Kontrastmittels repräsentiert, und aus mindestens einer nativen MRT-Aufnahme (MRI⁰), die die Leber des Untersuchungsobjekts ohne Kontrastmittel repräsentiert. Wie beschrieben ist die Verwendung von einer oder mehreren nativen MRT-Aufnahmen optional. Wie beschrieben können die Eingabedaten (I) weitere Daten umfassen, wobei die weiteren Daten jedoch keine MRT-Aufnahmen einer anderen Phase als die Übergangsphase und optional die native Phase umfassen. Die Zieldaten (T) bestehen im vorliegenden Beispiel aus einer zweiten MRT-Aufnahme (MRI¹), die die Leber zu einem Zeitpunkt in der hepatobiliären Phase nach der Applikation des Kontrastmittels repräsentiert.

Bei der mindestens einen ersten MRT-Aufnahme (MRI¹) kann es sich beispielsweise um die in Fig. 1 (e) gezeigte MRT-Aufnahme handeln. Bei der mindestens einen nativen MRT-Aufnahme (MRI⁰) kann es sich beispielsweise um die in Fig. 1 (a) gezeigte MRT-Aufnahme handeln. Bei der zweiten MRT-Aufnahme (MRI²) kann es sich beispielsweise um die in Fig. 1 (f) gezeigte MRT-Aufnahme handeln.

Die Eingabedaten (I) werden dem Modell des maschinellen Lernens (MLM) zugeführt. Das Modell des maschinellen Lernens (MLM) ist konfiguriert, auf Basis der Eingabedaten (I) und auf Basis von Modellparametern (MP) Ausgabedaten (O) zu erzeugen. Die Ausgabedaten (O) stellen eine vorhergesagte zweite MRT-Aufnahme (MRI^{2*}) dar, die die Leber des Untersuchungsobjekts zu einem Zeitpunkt in der Übergangsphase nach der Applikation eines hepatobiliären Kontrastmittels repräsentiert.

Mittels einer Fehlerfunktion (LF) werden Abweichungen zwischen der vorhergesagten zweiten MRT-Aufnahme (MRI^{2*}) und der zweiten MRT-Aufnahme (MRI²) der Trainingsdaten quantifiziert. Für jedes Paar aus Eingabedaten und Ausgabedaten kann ein Fehler (L) berechnet werden. Der Fehler (L) kann in einem Optimierungsverfahren (z.B. einem Gradientenverfahren) genutzt werden, um Modellparameter (MP) zu modifizieren, so dass der Fehler (L) auf ein definiertes Minimum reduziert wird.

Ist das Modell auf Basis einer Vielzahl an Paaren aus Eingabedaten und Zieldaten trainiert worden und hat der Fehler (L) für alle Paare ein definiertes Minimum erreicht, kann das trainierte Modell des maschinellen Lernens zur Vorhersage genutzt werden. Dies ist beispielhaft und schematisch in Fig. 4 gezeigt.

Fig. 4 zeigt beispielhaft und schematisch eine Ausführungsform des Verfahrens zur Vorhersage einer MRT-Aufnahme einer Leber oder eines Teils der Leber eines Patienten, wobei die MRT-Aufnahme die Leber oder den Teil der Leber in einer hepatobiliären Phase nach der Applikation eines hepatobiliären Kontrastmittels repräsentiert.

Die Vorhersage erfolgt mit Hilfe eines trainierten Modells (MLM^{t}) des maschinellen Lernens. Das hochgestellte "t" in MLM^{t} soll verdeutlichen, dass es sich um das trainierte Modell handelt. Das in Fig. 4 gezeigte trainierte Modell (MLM^{t}) des maschinellen Lernens kann beispielsweise wie in Bezug zu Fig. 3 beschrieben trainiert worden sein.

Die Vorhersage erfolgt auf Basis von Patientendaten (PD). Die Patientendaten (PD) umfassen mindestens eine erste MRT-Aufnahme (MRI_{P}¹), wobei die mindestens eine erste MRT-Aufnahme (MRI_{P}¹) die Leber oder den Teil der Leber des Patienten in der Übergangsphase nach der Applikation des hepatobiliären Kontrastmittels repräsentiert. Im vorliegenden Beispiel umfassen die Patientendaten (PD) ferner mindestens eine native MRT-Aufnahme (MRI_{P}⁰). Die mindestens eine native MRT-Aufnahme (MRI_{P}⁰) zeigt die Leber oder den Teil der Leber ohne ein Kontrastmittel.

Die MRT-Aufnahmen, die bei der Verwendung des trainierten Modells des maschinellen Lernens zur Vorhersage verwendet werden oder erzeugt werden, werden in dieser Beschreibung und in Fig. 4 mit einem tiefgestellten "P" gekennzeichnet, um sie von den MRT-Aufnahmen, die beim Trainieren des Modells des maschinellen Lernens verwendet werden oder erzeugt werden, zu unterscheiden.

Die Patientendaten (PD) können noch weitere Daten umfassen, insbesondere dann, wenn auch das Training des Modells des maschinellen Lernens auf Basis weiterer Eingabedaten erfolgt ist.

Die Patientendaten (PD) umfassen jedoch keine anderen/weiteren MRT-Aufnahmen als die mindestens eine erste MRT-Aufnahme und optional die mindestens eine native MRT-Aufnahme.

Die Patientendaten (PD) werden dem trainierten Modell (MLM^{t}) des maschinellen Lernens zugeführt. Das Modell (MLM^{t}) des maschinellen Lernens ist konfiguriert und trainiert, auf Basis der Patientendaten (PD) und auf Basis der im Rahmen des Trainings modifizierten (gelernten) Modellparametern (MP^{t}) eine zweite MRT-Aufnahme (MRI_{P}^{2*}) zu erzeugen, wobei die zweite MRT-Aufnahme (MRI_{P}^{2*}) eine vorhergesagte MRT-Aufnahme der Leber oder des Teils der Leber des Patienten in der hepatobiliären Phase nach der Applikation des hepatobiliären Kontrastmittels ist.

Die zweite MRT-Aufnahme (MRI_{P}^{2*}) kann auf einem Monitor angezeigt, auf einem Drucker ausgedruckt, auf einem Datenspeicher gespeichert und/oder an ein separates Computersystem übermittelt werden.

Das Modell des maschinellen Lernens kann beispielsweise ein künstliches neuronales Netz sein oder ein solches umfassen.

Ein künstliches neuronales Netzwerk umfasst mindestens drei Schichten von Verarbeitungselementen: eine erste Schicht mit Eingangsneuronen (Knoten), eine *N*-te Schicht mit mindestens einem Ausgangsneuron (Knoten) und *N*-2 innere Schichten, wobei *N* eine natürliche Zahl und größer als 2 ist.

Die Eingangsneuronen dienen zum Empfangen der Eingabedaten, insbesondere der mindestens einen ersten MRT-Aufnahme und ggf. der optionalen mindestens einen nativen MRT-Aufnahme. Beispielsweise kann es ein Eingangsneuron für jedes Pixel oder Voxel einer MRT-Aufnahme geben, wenn es sich bei der Repräsentation um eine Ortsraumdarstellung in Form einer Rastergrafik handelt. Es können zusätzliche Eingangsneuronen für zusätzliche Eingabedaten (z.B. Informationen zum Untersuchungsbereich, zum Untersuchungsobjekt, zu Bedingungen, die bei der Erzeugung der Repräsentation herrschten, Informationen zu dem Zustand, den die Repräsentation repräsentiert, und/oder Informationen zu dem Zeitpunkt oder der Zeitspanne zu/in der die Repräsentation erzeugt worden ist) vorhanden sein.

Die Ausgangsneuronen können dazu dienen, eine vorhergesagte zweite MRT-Aufnahme, die den Untersuchungsbereich zu einem Zeitpunkt in der hepatobiliären Phase repräsentiert, auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Vorhersage der zweiten MRT-Aufnahme angestrebt. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich des Zusammenhangs zwischen der mindestens einen ersten MRT-Aufnahme sowie optional der mindestens einen nativen MRT-Aufnahme und der zweiten MRT-Aufnahme, die für Vorhersagezwecke verwendet werden können.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netzwerk kann ein rekurrentes neuronales Netzwerk sein oder ein solches umfassen. Als rekurrente bzw. rückgekoppelte neuronale Netzwerke bezeichnet man neuronale Netzwerke, die sich im Gegensatz zu den Feedforward-Netzen durch Verbindungen von Neuronen einer Schicht zu Neuronen derselben oder einer vorangegangenen Schicht auszeichnen. Das künstliche neuronale Netzwerk kann beispielsweise ein *Long short-term memory* (LSTM) umfassen (siehe z.B. Y. Gao et al.: Fully convolutional structured LSTM networks for joint 4D medical image segmentation, DOI: 10.1109/ISBI.2018.8363764).

Das künstliche neuronale Netz kann ein Transfomer-Netzwerk sein (siehe z.B. D. Karimi et al.: Convolution-Free Medical Image Segmentation using Transformers, arXiv:2102.13645 [eess.IV]).

Die Erfindung kann ganz oder teilweise mit Hilfe eines Computersystems ausgeführt werden.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

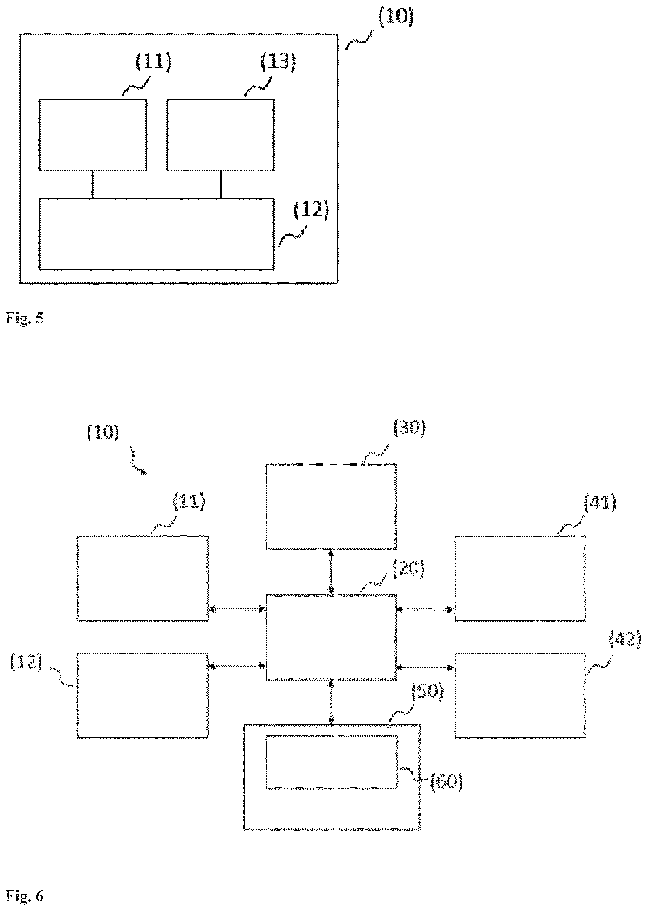

Fig. 5 zeigt beispielhaft und schematisch eine Ausführungsform des erfindungsgemäßen Computersystems.

Das in Fig. 5 gezeigte Computersystem (10) umfasst eine Eingabeeinheit (11), eine Steuer- und Recheneinheit (12) und eine Ausgabeeinheit (13).

Die Eingabeeinheit (11) dient dem Empfang von Daten (z.B. Patientendaten umfassend eine oder mehrere MRT-Aufnahmen) und/oder der Eingabe von Daten und/oder Befehlen durch einen Nutzer des Computersystems (10).

Die Ausgabeeinheit (13) dient der Ausgabe von Daten und/oder Informationen gegenüber einem Nutzer und/oder der Speicherung von Daten (z.B. der vorhergesagten MRT-Aufnahmen) in einem Datenspeicher und/oder der Übermittlung von Daten an ein separates Computersystem.

Die Steuer- und Recheneinheit (12) dient der Steuerung des Computersystems (10), der Koordinierung der Datenflüsse zwischen den Einheiten des Computersystems (10) und der Durchführung von Berechnungen.

Die Steuer- und Recheneinheit (12) ist konfiguriert
- die Eingabeeinheit (11) zu veranlassen, Patientendaten zu empfangen, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme die Leber oder den Teil der Leber des Patienten ohne Kontrastmittel repräsentiert,
- die Patientendaten in ein trainiertes Modell des maschinellen Lernens einzugeben, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel,
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- eine vorhergesagte MRT-Aufnahme von dem trainierten Modell des maschinellen Lernens zu empfangen, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- die Ausgabeeinheit (13) zu veranlassen, die vorhergesagte MRT-Aufnahme auszugeben und/oder zu speichern und/oder an ein separates Computersystem zu übermitteln.

Fig. 6 zeigt beispielhaft und schematisch eine weitere Ausführungsform des Computersystems. Das Computersystem (10) umfasst eine Verarbeitungseinheit (20), die mit einem Speicher (50) verbunden ist.

Die Verarbeitungseinheit (20) (engl.: *processing unit*) kann einen oder mehrere Prozessoren allein oder in Kombination mit einem oder mehreren Speichern umfassen. Bei der Verarbeitungseinheit (20) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (20) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (20) kann konfiguriert sein, Computerprogramme auszuführen, die in einem Arbeitsspeicher der Verarbeitungseinheit (20) oder im Speicher (50) desselben oder eines anderen Computersystems gespeichert sein können.

Der Speicher (50) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (50) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (50) kann die Verarbeitungseinheit (20) auch mit einer oder mehreren Schnittstellen (11, 12, 30, 41, 42) verbunden sein, um Informationen anzuzeigen, zu übertragen und/oder zu empfangen. Die Schnittstellen können eine oder mehrere Kommunikationsschnittstellen (11, 12) und/oder eine oder mehrere Benutzerschnittstellen (30, 41, 42) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (30) umfassen. Eine Anzeige (30) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (41, 42) kann/können verdrahtet oder drahtlos sein und kann/können so konfiguriert sein, dass sie Informationen von einem Benutzer in das Computersystem (10) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (60) können im Speicher (50) gespeichert sein und von der Verarbeitungseinheit (20) ausgeführt werden, die dadurch programmiert wird, die in dieser Beschreibung beschriebenen Funktionen zu erfüllen. Das Abrufen, Laden und Ausführen von Anweisungen des Computerprogramms (60) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (50) kann auch das erfindungsgemäße Modell des maschinellen Lernens gespeichert sein.

Das erfindungsgemäße Computersystem kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

Gegenstand der vorliegenden Erfindung ist auch ein Computerprogrammprodukt. Ein solches Computerprogrammprodukt umfasst einen nichtflüchtigen Datenträger wie beispielsweise eine CD, eine DVD, ein USB-Stick oder ein anderes Medium zum Speichern von Daten. Auf dem Datenträger ist ein Computerprogramm gespeichert. Das Computerprogramm kann in einen Arbeitsspeicher eines Computersystems (insbesondere in einen Arbeitsspeicher eines Computersystems der vorliegenden Offenbarung) geladen werden und dort das Computersystem dazu veranlassen, folgende Schritte ausführen:
- Empfangen von Patientendaten, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme die Leber oder den Teil der Leber des Patienten ohne Kontrastmittel repräsentiert,
- Eingeben der Patientendaten in ein trainiertes Modell des maschinellen Lernens, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen,
   wobei die Eingabedaten MRT-Aufnahmen umfassen, wobei die MRT-Aufnahmen beschränkt sind auf:
      ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
      ∘ optional mindestens eine native MRT-Aufnahme der Leber oder des Teils der Leber des Untersuchungsobjekts ohne Kontrastmittel
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
- Empfangen einer vorhergesagten MRT-Aufnahme von dem trainierten Modell des maschinellen Lernens, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
- Ausgeben und/oder Speichern der vorhergesagten MRT-Aufnahme und/oder Übermitteln der vorhergesagten MRT-Aufnahme an ein separates Computersystem.

Das Computerprogrammprodukt kann auch in Kombination (in einer Zusammenstellung) mit dem Kontrastmittel vermarktet werden. Eine solche Zusammenstellung wird auch als Kit bezeichnet. Ein solches Kit umfasst das Kontrastmittel und das Computerprogrammprodukt. Es ist auch möglich, dass ein solches Kit das Kontrastmittel und Mittel umfasst, die es einen Käufer erlauben, das Computerprogramm zu beziehen, z.B. von einer Internetseite herunterzuladen. Diese Mittel können einen Link, d.h. eine Adresse der Internetseite umfassen, auf der das Computerprogramm bezogen, z.B. von der das Computerprogramm auf ein mit dem Internet verbundenes Computersystem heruntergeladen werden kann. Diese Mittel können einen Code (z.B. eine alphanumerische Zeichenkette oder einen QR-Code, oder einen DataMatrix-Code oder einen Barcode oder eine anderen optisch und/oder elektronisch lesbaren Code) umfassen, mit dem der Käufer Zugang zu dem Computerprogramm erhält. Ein solcher Link und/oder Code kann beispielsweise auf einer Verpackung des Kontrastmittels aufgedruckt und/oder auf einem Beipackzettel zum Kontrastmittel aufgedruckt sein. Ein Kit ist somit ein Kombinationsprodukt umfassend ein Kontrastmittel und ein Computerprogramm (z.B. in Form eines Zugangs zu dem Computerprogramm oder in Form von ausführbarem Programmcode auf einem Datenträger), das zusammen zum Kauf angeboten wird.

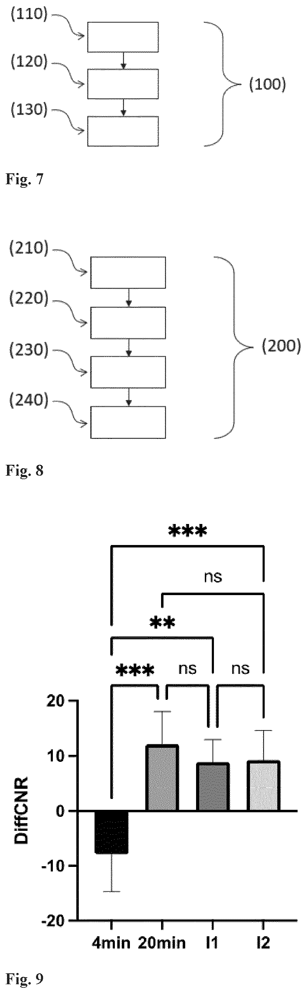

Fig. 7 zeigt eine Ausführungsform des Verfahrens zum Trainieren des Modells des maschinellen Lernens in Form eines Ablaufschemas. Das Verfahren (100) umfasst die Schritte:
(110) Empfangen und/oder Bereitstellen von Trainingsdaten, wobei die Trainingsdaten für jedes Untersuchungsobjekt einer Vielzahl an Untersuchungsobjekten Eingabedaten und Zieldaten umfassen, wobei die Eingabedaten MRT-Aufnahmen umfassen, wobei die MRT-Aufnahmen beschränkt sind auf:
   ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
   o optional mindestens eine native MRT-Aufnahme der Leber oder des Teils der Leber des Untersuchungsobjekts ohne Kontrastmittel,
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
(120) Trainieren des Modells des maschinellen Lernens, wobei das Modell des maschinellen Lernens konfiguriert ist, auf Basis mindestens einer ersten MRT-Aufnahme, optional mindestens einer nativen MRT-Aufnahme und Modellparametern eine vorhergesagte zweite MRT-Aufnahme zu erzeugen, wobei das Trainieren für jedes Untersuchungsobjekt der Vielzahl an Untersuchungsobjekten umfasst:
   ∘ Eingeben der Eingabedaten in das Modell des maschinellen Lernens,
   ∘ Empfangen einer vorhergesagten zweiten MRT-Aufnahme von dem Modell des maschinellen Lernens,
   ∘ Berechnen einer Abweichung zwischen der zweiten MRT-Aufnahme und der vorhergesagten zweiten MRT-Aufnahme,
   ∘ Modifizieren der Modellparameter im Hinblick auf eine Reduzierung der Abweichung,
(130) Speichern und/oder Ausgeben des trainierten Modells des maschinellen Lernens und/oder Übermitteln des trainierten Modells des maschinellen Lernens auf ein separates Computersystem und/oder Verwenden des trainierten Modells des maschinellen Lernens zur Vorhersage.

Fig. 8 zeigt eine Ausführungsform des computer-implementierten Verfahrens zum Verwenden des trainierten Modells des maschinellen Lernens zur Vorhersage einer MRT-Aufnahme in Form eines Ablaufschemas. Das Verfahren (200) umfasst die Schritte:
(210) Empfangen von Patientendaten, wobei die Patientendaten mindestens eine MRT-Aufnahme umfassen, wobei die mindestens eine MRT-Aufnahme beschränkt ist auf mindestens eine erste MRT-Aufnahme und optional mindestens eine native MRT-Aufnahme, wobei die mindestens eine erste MRT-Aufnahme eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme die Leber oder den Teil der Leber des Patienten ohne Kontrastmittel repräsentiert,
(220) Eingeben der Patientendaten in ein trainiertes Modell des maschinellen Lernens, wobei das Modell des maschinellen Lernens anhand von Trainingsdaten trainiert wurde, auf Basis mindestens einer ersten MRT-Aufnahme und optional mindestens einer nativen MRT-Aufnahme eine zweite MRT-Aufnahme vorherzusagen, wobei die Trainingsdaten für jedes Untersuchungsobjekt einer Vielzahl an Untersuchungsobjekten Eingabedaten und Zieldaten umfassen, wobei die Eingabedaten MRT-Aufnahmen umfassen,
   wobei die MRT-Aufnahmen beschränkt sind auf:
   ∘ mindestens eine erste MRT-Aufnahme, wobei die mindestens eine erste MRT-Aufnahme eine Leber oder einen Teil der Leber des Untersuchungsobjekts zu einem Zeitpunkt in der Übergangsphase nach einer Applikation eines hepatobiliären Kontrastmittels repräsentiert, und
   ∘ optional mindestens eine native MRT-Aufnahme der Leber oder des Teils der Leber des Untersuchungsobjekts ohne Kontrastmittel,
   wobei die Zieldaten eine zweite MRT-Aufnahme umfassen, wobei die zweite MRT-Aufnahme die Leber oder den Teil der Leber des Untersuchungsobjekts zu einem Zeitpunkt in der hepatobiliären Phase nach der Applikation des hepatobiliären Kontrastmittels repräsentiert,
(230) Empfangen einer vorhergesagten MRT-Aufnahme von dem trainierten Modell des maschinellen Lernens, wobei die vorhergesagte MRT-Aufnahme die Leber oder den Teil der Leber des Patienten zu einem Zeitpunkt in der hepatobiliären Phase nach der Applikation des hepatobiliären Kontrastmittels repräsentiert,
(240) Ausgeben und/oder Speichern der vorhergesagten MRT-Aufnahme und/oder Übermitteln der vorhergesagten MRT-Aufnahme an ein separates Computersystem.

### Beispiel

Im Folgenden wird eine Beispielimplementierung (11) zur Reduzierung der erforderlichen Wartezeit der Akquise einer MRT-Aufnahme der Leber eines Untersuchungsobjekts in der hepatobiliären Phase beschrieben. Die MRT-Aufnahme wird auf Basis mindestens einer ersten MRT-Aufnahme, die die Leber in der Übergangsphase repräsentiert und mindestens einer nativen MRT-Aufnahme vorhergesagt.

Die mindestens eine erste MRT-Aufnahme wurde 4 Minuten nach Applikation eines hepatobiliären Kontrastmittels (Primovist^{®}) aufgezeichnet.

Die Ziel-MRT-Aufnahme (*target, ground truth*) wurde 20 Minuten nach Applikation des hepatobiliären Kontrastmittels aufgezeichnet.

Die Beispielimplementierung (I1) wird mit einer zweiten Implementierung (I2) vergleichen, bei welcher neben der mindestens einen ersten MRT-Aufnahme und der mindestens einen nativen MRT-Aufnahme weitere MRT-Aufnahmen aus weiteren Phasen in die Vorhersage der MRT-Aufnahme der Leber des Untersuchungsobjekts in der hepatobiliären Phase einbezogen wurden.

Die weiteren MRT-Aufnahmen wurden 18 Sekunden (arterielle Phase), 65 Sekunden (portal-venöse Phase) und 95 Sekunden (spätvenöse Phase) nach Applikation des hepatobiliären Kontrastmittels aufgezeichnet.

Zur Gewinnung eines Trainingsdatensatzes wurden 20 gesunde Göttinger Minischweine bei drei verschiedenen Gelegenheiten einer MRT mit einer Standarddosis (0,025mmol/kg, Gadoxetat-Dinatrium) unterzogen. Drei von 19 Tieren wurden nach dem Zufallsprinzip ausgewählt und für die Validierung zurückgehalten (18 Untersuchungen). Ein Tier musste wegen unvollständiger Untersuchungen ausgeschlossen werden. Die verbleibenden 16 Tiere (96 Untersuchungen) wurden verwendet, um ein GAN (CycleGAN) für eine Bild-zu-Bild-Umwandlung zu trainieren.

In der Beispielimplementierung (I1) wurden drei Schichten in den Lebern der Tiere betrachtet: X1, X2, X3. Es wurden erste MRT-Aufnahmen und native Aufnahmen der Schichten X1, X2, X3 als Eingabedaten verwendet, um das Ziel Y vorherzusagen.

Das vorherzusagende Ziel Y befindet sich an der gleichen Position wie die Schicht X2.

Das vorherzusagende Ziel Y ist eine Wasserkarte einer T1-VIBE-DIXON-Sequenz.

Für die Schicht X2, die Schicht X1 (oberhalb von X2) und die Schicht X3 (unterhalb von X2) wurden drei Stapel von jeweils fünf MRT-Aufnahmen erzeugt. Jeder der Bildstapel enthält die Wasserkarte, die Fettkarte, die In-Phase und die Opposed-Phase einer T1-VIBE-DIXON-Sequenz, die 4 Minuten nach Injektion eines leberspezifischen Kontrastmittels (0,025 mmol/kg, Gadoxetat) aufgezeichnet wurde und eine Wasserkarte einer T1-VIBE-DIXON, die vor der Injektion aufgenommen wurde (insgesamt 15 MRT-Aufnahmen).

Als vergleichende Implementierung (I2) wurden zusätzlich die Wasserkarte einer arteriellen, portalvenösen und spätvenösen T1-VIBE-DIXON-Sequenz der Schichten X1, X2, X3 eingegeben. Dies resultiert in einer Gesamtzahl von 24 MRT-Aufnahmen. Alle übrigen Einstellungen entsprechen der Implementierung I1. Für die Akquisition der T1-VIBE-DIXON-Sequenz wurden an einem 1,5T Avanto Fit MRT Scanner (Siemens Healthcare GmbH) folgende Scanparameter verwendet: TR 6,9 ms, TE 2,39/4,77 ms, Flippwinkel 10°, Matrix 256x256, Schichtdicke 1,5mm.

Die Modelle des maschinellen Lernens der beiden Implementierungen (jeweils CycleGAN) wurden mit 200 Epochen und einem linearen Lernratenabfall, beginnend nach 100 Epochen, trainiert. Die anfängliche Lernrate wurde auf 0,0002 festgelegt, und es wurde ein Momentum von 0,5 verwendet. Außerdem wurde für jedes Modell eine Batchgröße von 4 verwendet. Was die Netzwerkarchitektur betrifft, so wurde ein ResNet 9 Block-Generator mit 64 Filtern in der letzten Faltungsschicht, Transpositionsfaltung als Upsampling-Methode und Instanznormalisierung verwendet. Darüber hinaus wurde ein PatchGAN als Diskriminator eingesetzt, der 64 Filter in der ersten Faltungsschicht enthält. Während des Trainings wurde eine ausführliche Datenaugmentation verwendet, darunter zufälliges Zuschneiden (224 x 224), zufällige Rotation (-15 bis 15 Grad) und horizontales Spiegeln. Um die Gesamtkompatibilität innerhalb jedes Datenstapels zu gewährleisten, wurden alle MRT-Aufnahmen auf eine Größe von 224 x 224 verkleinert, um der zufälligen Zuschnittsgröße zu entsprechen.

Zur Validierung der Effizienz des Netzes wurden ROI-Messungen in der abdominellen Aorta, der inferioren Vena cava, der Pfortader, dem Leberparenchym und der autochthonen Rückenmuskulatur durchgeführt und das Kontrast-Rausch-Verhältnis (CNR) berechnet ((Intensität Zielstruktur - Intensität autochthone Rückenmuskulatur) / Standardabweichung der Intensität der Rückenmuskulatur).

Unter Annahme, dass vom Zeitpunkt TP4 (siehe Figur 2) zum zu vorhersagenden Zeitpunkt TP5 (siehe Figur 2) das vaskuläre CNR abgesenkt und das parenchymale CNR angehoben werden muss, wurde als Bewertungsmetrik die Differenz des hepatischen CNR zum Durchschnitt des vaskulären CNR berechnet (DiffCNR).

Hier zeigte sich ein signifikanter Anstieg des DiffCNR der Übergangsphase (4 Minuten nach Kontrastmittelgabe) zur echten hepatobiliären Kontrastmittelphase (Durchschnittsdifferenz = -19,96; p = 0.0005) und zur vorhergesagten MRT-Aufnahme der Implementierung I1 (Durchschnittsdifferenz = - 16,76; p = 0.0012) und I2 (Durchschnittsdifferenz = -17,14; p =0.0003).

Es zeigte sich kein signifikanter Unterschied zwischen dem DiffCNR der echten hepatobilären Kontrastmittelphase und den vorhergesagten MRT-Aufnahmen der Implementierung I1 und I2. Es zeigten sich zudem keine signifikanten Unterschiede zwischen den vorhergesagten MRT-Aufnahmen der Implementierung I1 und I2.

In Fig. 9 sind die Ergebnisse der DiffCNR in Form einer Grafik Messungen dargestellt. In der Grafik sind die arithmetischen Mittelwerte und Standardabweichungen der DiffCNR-Werte für die Implementierungen I1 und I2 dargestellt. ns bedeutet "nicht signifikant". ** steht für p < 0,01. *** steht für p < 0,001.

Es konnte gezeigt werden, dass zur Erzeugung einer MRT-Aufnahme der Leber oder eines Teils der Leber eines Untersuchungsobjekts mindestens eine MRT-Aufnahme der Leber oder des Teils der Leber in der Übergangsphase und optional mindestens eine native MRT-Aufnahme der Leber oder des Teils der Leber ausreichend ist.

Durch die Reduzierung der Zahl an MRT-Aufnahmen sind weniger Ressourcen nötig. Zur Erzeugung von Trainingsdaten müssen weniger MRT-Aufnahmen erzeugt werden. Das Trainieren des Modells des maschinellen Lernens erfolgt schneller und benötigt weniger Datenspeicher und Computerleistung. Dies gilt analog auch für die Nutzung des trainierten Modells des maschinellen Lernens für die Vorhersage. Dadurch, dass für das Trainieren und die Vorhersage nur zu maximal zwei Zeitpunkten MRT-Aufnahmen erzeugt werden, ist die Erzeugung der MRT-Aufnahmen für die Untersuchungsobjekte weniger unangenehm. Sie müssen nur zu maximal zwei Zeitpunkten Bewegungen einstellen (um Bewegungsartefakte zu vermeiden). Dabei liegen die zwei Zeitpunkt so weit auseinander (mehr als 3 Minuten), dass sie zwischen diesen Zeitpunkten entspannen können.

## Patentansprüche

1. Computer-implementiertes Verfahren umfassend:
• Bereitstellen eines trainierten Modells (MLM^{t}) des maschinellen Lernens, wobei das trainierte Modell (MLM^{t}) des maschinellen Lernens anhand von Trainingsdaten (TD) trainiert wurde, auf Basis mindestens einer ersten MRT-Aufnahme (MRI¹) und optional mindestens einer nativen MRT-Aufnahme (MRI⁰) eine vorhergesagte zweite MRT-Aufnahme (MRI^{2*}) zu erzeugen, wobei die Trainingsdaten (TD) für jedes Untersuchungsobjekt einer Vielzahl an Untersuchungsobjekten Eingabedaten (I) und Zieldaten (T) umfassen,
wobei die Eingabedaten (I) MRT-Aufnahmen (MRI⁰, MRI¹) umfassen, wobei die MRT-Aufnahmen (MRI⁰, MRI¹) beschränkt sind auf:
∘ mindestens eine erste MRT-Aufnahme (MRI¹), wobei die mindestens eine erste MRT-Aufnahme (MRI¹) eine Leber oder einen Teil der Leber des Untersuchungsobjekts zu einem Zeitpunkt in der Übergangsphase nach einer Applikation eines hepatobiliären Kontrastmittels repräsentiert, und
∘ optional mindestens eine native MRT-Aufnahme (MRI⁰) der Leber oder des Teils der Leber des Untersuchungsobjekts ohne Kontrastmittel,
wobei die Zieldaten (T) eine zweite MRT-Aufnahme (MRI²) umfassen, wobei die zweite MRT-Aufnahme (MRI²) die Leber oder den Teil der Leber des Untersuchungsobjekts zu einem Zeitpunkt in der hepatobiliären Phase nach der Applikation des hepatobiliären Kontrastmittels repräsentiert,
• Empfangen von Patientendaten (PD), wobei die Patientendaten (PD) mindestens eine MRT-Aufnahme (MRI_{P}⁰, MRI_{P}¹) umfassen, wobei die mindestens eine MRT-Aufnahme (MRI_{P}⁰, MRI_{P}¹) beschränkt ist auf mindestens eine erste MRT-Aufnahme (MRI_{P}¹) und optional mindestens eine native MRT-Aufnahme (MRI_{P}⁰), wobei die mindestens eine erste MRT-Aufnahme (MRI_{P}¹) eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme (MRI_{P}⁰) die Leber oder den Teil der Leber des Patienten ohne Kontrastmittel repräsentiert,
• Eingeben der Patientendaten (PD) in das trainierte Modell (MLM^{t}) des maschinellen Lernens,
• Empfangen einer vorhergesagten MRT-Aufnahme (MRI_{P}^{2*}) von dem trainierten Modell (MLM^{t}) des maschinellen Lernens, wobei die vorhergesagte MRT-Aufnahme (MRI_{P}^{2*}) die Leber oder den Teil der Leber des Patienten zu einem Zeitpunkt in der hepatobiliären Phase nach der Applikation des hepatobiliären Kontrastmittels repräsentiert,
• Ausgeben und/oder Speichern der vorhergesagten MRT-Aufnahme (MRI_{P}²*) und/oder Übermitteln der vorhergesagten MRT-Aufnahme (MRI_{P}²*) an ein separates Computersystem.

2. Verfahren gemäß Anspruch 1, wobei das Trainieren des Modells (MLM^{t}) des maschinellen Lernens umfasst:
• Empfangen und/oder Bereitstellen der Trainingsdaten (TD),
• Trainieren des Modells (MLM) des maschinellen Lernens, wobei das Modell (MLM) des maschinellen Lernens konfiguriert ist, auf Basis mindestens einer ersten MRT-Aufnahme (MRI¹), optional mindestens einer nativen MRT-Aufnahme (MRI⁰) und Modellparametern (MP) eine vorhergesagte zweite MRT-Aufnahme (MRI^{2*}) zu erzeugen, wobei das Trainieren für jedes Untersuchungsobjekt der Vielzahl an Untersuchungsobjekten umfasst:
∘ Eingeben der Eingabedaten (I) in das Modell (MLM) des maschinellen Lernens,
∘ Empfangen einer vorhergesagten zweiten MRT-Aufnahme (MRI^{2*}) von dem Modell (MLM) des maschinellen Lernens,
∘ Berechnen einer Abweichung zwischen der zweiten MRT-Aufnahme (MRI²) und der vorhergesagten zweiten MRT-Aufnahme (MRI^{2*}),
∘ Modifizieren der Modellparameter (MP) im Hinblick auf eine Reduzierung der Abweichung,
• Speichern und/oder Ausgeben des trainierten Modells (MLM^{t}) des maschinellen Lernens und/oder Übermitteln des trainierten Modells (MLM^{t}) des maschinellen Lernens an ein separates Computersystem und/oder Verwenden des trainierten Modells (MLM^{t}) des maschinellen Lernens zur Vorhersage.

3. Verfahren gemäß Anspruch 1 oder 2, wobei das Untersuchungsobjekt ein Mensch ist.

4. Verfahren gemäß einem der Ansprüche 1 bis 3, wobei die mindestens eine erste MRT-Aufnahme (MRI_{P}¹, MRI¹) die Leber oder den Teil der Leber des Patienten/Untersuchungsobjekts 3 bis 6 Minuten nach der Applikation des Kontrastmittels repräsentiert.

5. Verfahren gemäß einem der Ansprüche 1 bis 4, wobei die mindestens eine erste MRT-Aufnahme (MRI_{P}¹, MRI¹) mindestens eine T1-gewichtete MRT-Aufnahme ist oder eine solche umfasst.

6. Verfahren gemäß einem der Ansprüche 1 bis 5, wobei die mindestens eine erste MRT-Aufnahme (MRI_{P}¹, MRI¹) das Ergebnis einer Dixon-Sequenz, vorzugweise einer T1-gewichteten Dixon-Sequenz ist.

7. Verfahren gemäß einem der Ansprüche 1 bis 6, wobei die mindestens eine erste MRT-Aufnahme (MRI_{P}¹, MRI¹) eine In-Phase-Aufnahme und/oder eine Opposed-Phase-Aufnahme und/oder eine Fettkarte und/oder eine Wasserkarte der Leber oder des Teils der Leber umfasst.

8. Verfahren gemäß einem der Ansprüche 1 bis 7, wobei die Eingabedaten (I) und/oder Patientendaten (PD) mindestens eine native MRT-Aufnahme (MRI⁰, MRI_{P}⁰) umfassen, wobei die mindestens eine native MRT-Aufnahme (MRI⁰, MRI_{P}⁰) das Ergebnis einer Dixon-Sequenz, vorzugweise einer T1-gewichteten Dixon-Sequenz, vorzugsweise mit einer In- und Opposed-Phase Aufnahmetechnik ist.

9. Verfahren gemäß einem der Ansprüche 1 bis 8, wobei die zweite MRT-Aufnahme (MRI_{P}², MRI²) eine Repräsentation der Leber zu einem Zeitpunkt im Bereich von 10 Minuten bis 30 Minuten, vorzugsweise 15 Minuten bis 25 Minuten nach der Applikation des hepatobiliären Kontrastmittels ist.

10. Verfahren gemäß einem der Ansprüche 1 bis 9, wobei die zweite MRT-Aufnahme (MRI_{P}², MRI²) eine T1-gewichtete MRT-Aufnahme, vorzugsweise eine Wasserkarte ist.

11. Verfahren gemäß einem der Ansprüche 1 bis 10, wobei das Kontrastmittel umfasst:
- das Dinatriumsalz der Gadoxetsäure,
- Gadolinium 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) oder
- Gadolinium 2,2',2"-{10-[(1S)-4-(4-butoxyphenyl)-1-carboxybutyl]-1,4,7,10-tetraazacyclododecan-1,4,7-triyl}triacetat.

12. Computersystem (10) umfassend
• eine Eingabeeinheit (11),
• eine Steuer- und Recheneinheit (12) und
• eine Ausgabeeinheit (13),
wobei die Steuer- und Recheneinheit (12) konfiguriert ist,
• die Eingabeeinheit (11) zu veranlassen, Patientendaten (PD) zu empfangen, wobei die Patientendaten (PD) mindestens eine MRT-Aufnahme (MRI_{P}⁰, MRI_{P}¹) umfassen, wobei die mindestens eine MRT-Aufnahme (MRI_{P}⁰, MRI_{P}¹) beschränkt ist auf mindestens eine erste MRT-Aufnahme (MRI_{P}¹) und optional mindestens eine native MRT-Aufnahme (MRI_{P}⁰), wobei die mindestens eine erste MRT-Aufnahme (MRI_{P}¹) eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme (MRI_{P}⁰) die Leber oder den Teil der Leber des Patienten ohne Kontrastmittel repräsentiert,
• die Patientendaten (PD) in ein trainiertes Modell (MLM^{t}) des maschinellen Lernens einzugeben, wobei das Modell (MLM^{t}) des maschinellen Lernens anhand von Trainingsdaten (TD) trainiert wurde, auf Basis mindestens einer ersten MRT-Aufnahme (MRI¹) und optional mindestens einer nativen MRT-Aufnahme (MRI⁰) eine vorhergesagte zweite MRT-Aufnahme (MRI^{2*}) zu erzeugen, wobei die Trainingsdaten (TD) für jedes Untersuchungsobjekt einer Vielzahl an Untersuchungsobjekten Eingabedaten (I) und Zieldaten (T) umfassen, wobei die Eingabedaten (I) MRT-Aufnahmen (MRI⁰, MRI¹) umfassen,
wobei die MRT-Aufnahmen (MRI⁰, MRI¹) beschränkt sind auf:
∘ mindestens eine erste MRT-Aufnahme (MRI¹), wobei die mindestens eine erste MRT-Aufnahme (MRI¹) eine Leber oder einen Teil der Leber des Untersuchungsobjekts zu einem Zeitpunkt in der Übergangsphase nach einer Applikation eines hepatobiliären Kontrastmittels repräsentiert, und
∘ optional mindestens eine native MRT-Aufnahme (MRI⁰) der Leber oder des Teils der Leber des Untersuchungsobjekts ohne Kontrastmittel,
wobei die Zieldaten (T) eine zweite MRT-Aufnahme (MRI²) umfassen, wobei die zweite MRT-Aufnahme (MRI²) die Leber oder den Teil der Leber des Untersuchungsobjekts zu einem Zeitpunkt in der hepatobiliären Phase nach der Applikation des hepatobiliären Kontrastmittels repräsentiert,
• eine vorhergesagte MRT-Aufnahme (MRI_{P}^{2*}) von dem trainierten Modell (MLM^{t}) des maschinellen Lernens zu empfangen, wobei die vorhergesagte MRT-Aufnahme (MRI_{P}^{2*}) die Leber oder den Teil der Leber des Patienten zu einem Zeitpunkt in der hepatobiliären Phase nach der Applikation des hepatobiliären Kontrastmittels repräsentiert,
• die Ausgabeeinheit (13) zu veranlassen, die vorhergesagte MRT-Aufnahme (MRI_{P}^{2*}) auszugeben und/oder zu speichern und/oder an ein separates Computersystem zu übermitteln.

13. Computerprogrammprodukt umfassend einen Datenspeicher, in dem ein Computerprogramm (60) gespeichert ist, das in einen Arbeitsspeicher (50) eines Computersystems (10) geladen werden kann und dort das Computersystem (10) dazu veranlasst, folgende Schritte ausführen:
• Empfangen von Patientendaten (PD), wobei die Patientendaten (PD) mindestens eine MRT-Aufnahme (MRI_{P}⁰, MRI_{P}¹) umfassen, wobei die mindestens eine MRT-Aufnahme (MRI_{P}⁰, MRI_{P}¹) beschränkt ist auf mindestens eine erste MRT-Aufnahme (MRI_{P}¹) und optional mindestens eine native MRT-Aufnahme (MRI_{P}⁰), wobei die mindestens eine erste MRT-Aufnahme (MRI_{P}¹) eine Leber oder einen Teil der Leber eines Patienten zu einem Zeitpunkt in der Übergangsphase nach einer Applikation eines hepatobiliären Kontrastmittels repräsentiert, wobei die optionale mindestens eine native MRT-Aufnahme (MRI_{P}⁰) die Leber oder den Teil der Leber des Patienten ohne Kontrastmittel repräsentiert,
• Eingeben der Patientendaten (PD) in ein trainiertes Modell (MLM^{t}) des maschinellen Lernens, wobei das Modell (MLM^{t}) des maschinellen Lernens anhand von Trainingsdaten (TD) trainiert wurde, auf Basis mindestens einer ersten MRT-Aufnahme (MRI¹) und optional mindestens einer nativen MRT-Aufnahme (MRI⁰) eine vorhergesagte zweite MRT-Aufnahme (MRI^{2*}) zu erzeugen, wobei die Trainingsdaten (TD) für jedes Untersuchungsobjekt einer Vielzahl an Untersuchungsobjekten Eingabedaten (I) und Zieldaten (T) umfassen, wobei die Eingabedaten (I) MRT-Aufnahmen (MRI⁰, MRI¹) umfassen,
wobei die MRT-Aufnahmen (MRI⁰, MRI¹) beschränkt sind auf:
∘ mindestens eine erste MRT-Aufnahme (MRI¹), wobei die mindestens eine erste MRT-Aufnahme (MRI¹) eine Leber oder einen Teil der Leber des Untersuchungsobjekts zu einem Zeitpunkt in der Übergangsphase nach einer Applikation eines hepatobiliären Kontrastmittels repräsentiert, und
∘ optional mindestens eine native MRT-Aufnahme (MRI⁰) der Leber oder des Teils der Leber des Untersuchungsobjekts ohne Kontrastmittel,
wobei die Zieldaten (T) eine zweite MRT-Aufnahme (MRI²) umfassen, wobei die zweite MRT-Aufnahme (MRI²) die Leber oder den Teil der Leber des Untersuchungsobjekts zu einem Zeitpunkt in der hepatobiliären Phase nach der Applikation des hepatobiliären Kontrastmittels repräsentiert,
• Empfangen einer vorhergesagten MRT-Aufnahme (MRI_{P}²*) von dem trainierten Modell (MLM^{t}) des maschinellen Lernens, wobei die vorhergesagte MRT-Aufnahme (MRI_{P}^{2*}) die Leber oder den Teil der Leber des Patienten zu einem Zeitpunkt in der hepatobiliären Phase nach der Applikation des hepatobiliären Kontrastmittels repräsentiert,
• Ausgeben und/oder Speichern der vorhergesagten MRT-Aufnahme (MRI_{P}^{2*}) und/oder Übermitteln der vorhergesagten MRT-Aufnahme (MRI_{P}^{2*}) an ein separates Computersystem.

14. Verwendung eines hepatobiliären Kontrastmittels in einem MRT-Untersuchungsverfahren umfassend:
• Empfangen und/oder Erzeugen von Patientendaten (PD), wobei die Patientendaten (PD) mindestens eine MRT-Aufnahme (MRI_{P}⁰, MRI_{P}¹) umfassen, wobei die mindestens eine MRT-Aufnahme (MRI_{P}⁰, MRI_{P}¹) beschränkt ist auf mindestens eine erste MRT-Aufnahme (MRI_{P}¹) und optional mindestens eine native MRT-Aufnahme (MRI_{P}⁰), wobei die mindestens eine erste MRT-Aufnahme (MRI_{P}¹) eine Leber oder einen Teil der Leber eines Patienten zu einem Zeitpunkt in der Übergangsphase nach einer Applikation des hepatobiliären Kontrastmittels repräsentiert, wobei die optionale mindestens eine native MRT-Aufnahme (MRI_{P}⁰) die Leber oder den Teil der Leber des Patienten ohne Kontrastmittel repräsentiert,
• Eingeben der Patientendaten (PD) in ein trainiertes Modell (MLM^{t}) des maschinellen Lernens, wobei das Modell (MLM^{t}) des maschinellen Lernens anhand von Trainingsdaten (TD) trainiert wurde, auf Basis mindestens einer ersten MRT-Aufnahme (MRI¹) und optional mindestens einer nativen MRT-Aufnahme (MRI⁰) eine vorhergesagte zweite MRT-Aufnahme (MRI^{2*}) zu erzeugen, wobei die Trainingsdaten (TD) für jedes Untersuchungsobjekt einer Vielzahl an Untersuchungsobjekten Eingabedaten (I) und Zieldaten (T) umfassen, wobei die Eingabedaten (I) MRT-Aufnahmen (MRI⁰, MRI¹) umfassen,
wobei die MRT-Aufnahmen (MRI⁰, MRI¹) beschränkt sind auf:
∘ mindestens eine erste MRT-Aufnahme (MRI¹), wobei die mindestens eine erste MRT-Aufnahme (MRI¹) eine Leber oder einen Teil der Leber des Untersuchungsobjekts zu einem Zeitpunkt in der Übergangsphase nach einer Applikation eines hepatobiliären Kontrastmittels repräsentiert, und
∘ optional mindestens eine native MRT-Aufnahme (MRI⁰) der Leber oder des Teils der Leber des Untersuchungsobjekts ohne Kontrastmittel,
wobei die Zieldaten (T) eine zweite MRT-Aufnahme (MRI²) umfassen, wobei die zweite MRT-Aufnahme (MRI²) die Leber oder den Teil der Leber des Untersuchungsobjekts zu einem Zeitpunkt in der hepatobiliären Phase nach der Applikation des hepatobiliären Kontrastmittels repräsentiert,
• Empfangen einer vorhergesagten MRT-Aufnahme (MRI_{P}^{2*}) von dem trainierten Modell (MLM^{t}) des maschinellen Lernens, wobei die vorhergesagte MRT-Aufnahme (MRI_{P}^{2*}) die Leber oder den Teil der Leber des Patienten zu einem Zeitpunkt in der hepatobiliären Phase nach der Applikation des hepatobiliären Kontrastmittels repräsentiert,
• Ausgeben und/oder Speichern der vorhergesagten MRT-Aufnahme (MRI_{P}^{2*}) und/oder Übermitteln der vorhergesagten MRT-Aufnahme (MRI_{P}^{2*}) an ein separates Computersystem.

15. Kit umfassend ein hepatobiliäres Kontrastmittel und ein Computerprogrammprodukt, wobei das Computerprogrammprodukt einen Datenspeicher umfasst, in dem ein Computerprogramm (60) gespeichert ist, das in einen Arbeitsspeicher (50) eines Computersystems (10) geladen werden kann und dort das Computersystem (10) dazu veranlasst, folgende Schritte ausführen:
• Empfangen von Patientendaten (PD), wobei die Patientendaten (PD) mindestens eine MRT-Aufnahme (MRI_{P}⁰, MRI_{P}¹) umfassen, wobei die mindestens eine MRT-Aufnahme (MRI_{P}⁰, MRI_{P}¹) beschränkt ist auf mindestens eine erste MRT-Aufnahme (MRI_{P}¹) und optional mindestens eine native MRT-Aufnahme (MRI_{P}⁰), wobei die mindestens eine erste MRT-Aufnahme (MRI_{P}¹) eine Leber oder einen Teil der Leber eines Patienten zu einem Zeitpunkt in der Übergangsphase nach einer Applikation des hepatobiliären Kontrastmittels repräsentiert, wobei die optionale mindestens eine native MRT-Aufnahme (MRI_{P}⁰) die Leber oder den Teil der Leber des Patienten ohne Kontrastmittel repräsentiert,
• Eingeben der Patientendaten (PD) in ein trainiertes Modell (MLM^{t}) des maschinellen Lernens, wobei das Modell (MLM^{t}) des maschinellen Lernens anhand von Trainingsdaten (TD) trainiert wurde, auf Basis mindestens einer ersten MRT-Aufnahme (MRI¹) und optional mindestens einer nativen MRT-Aufnahme (MRI⁰) eine vorhergesagte zweite MRT-Aufnahme (MRI^{2*}) zu erzeugen, wobei die Trainingsdaten (TD) für jedes Untersuchungsobjekt einer Vielzahl an Untersuchungsobjekten Eingabedaten (I) und Zieldaten (T) umfassen, wobei die Eingabedaten (I) MRT-Aufnahmen (MRI⁰, MRI¹) umfassen,
wobei die MRT-Aufnahmen (MRI⁰, MRI¹) beschränkt sind auf:
∘ mindestens eine erste MRT-Aufnahme (MRI¹), wobei die mindestens eine erste MRT-Aufnahme (MRI¹) eine Leber oder einen Teil der Leber des Untersuchungsobjekts zu einem Zeitpunkt in der Übergangsphase nach einer Applikation eines hepatobiliären Kontrastmittels repräsentiert, und
∘ optional mindestens eine native MRT-Aufnahme (MRI⁰) der Leber oder des Teils der Leber des Untersuchungsobjekts ohne Kontrastmittel,
wobei die Zieldaten (T) eine zweite MRT-Aufnahme (MRI²) umfassen, wobei die zweite MRT-Aufnahme (MRI²) die Leber oder den Teil der Leber des Untersuchungsobjekts zu einem Zeitpunkt in der hepatobiliären Phase nach der Applikation des hepatobiliären Kontrastmittels repräsentiert,
• Empfangen einer vorhergesagten MRT-Aufnahme (MRI_{P}^{2*}) von dem trainierten Modell (MLM^{t}) des maschinellen Lernens, wobei die vorhergesagte MRT-Aufnahme (MRI_{P}^{2*}) die Leber oder den Teil der Leber des Patienten zu einem Zeitpunkt in der hepatobiliären Phase nach der Applikation des hepatobiliären Kontrastmittels repräsentiert,
• Ausgeben und/oder Speichern der vorhergesagten MRT-Aufnahme (MRI_{P}^{2*}) und/oder Übermitteln der vorhergesagten MRT-Aufnahme (MRI_{P}^{2*}) an ein separates Computersystem.

## Claims

1. Computer-implemented method, comprising:
• providing a trained machine learning model (MLM^{t}), wherein the trained machine learning model (MLM^{t}) was trained on the basis of training data (TD) to generate a predicted second MRI image (MRI^{2*}) on the basis of at least one first MRI image (MRI¹) and optionally at least one native MRI image (MRI⁰), wherein the training data (TD) comprise input data (I) and target data (T) for each examination object of a plurality of examination objects, wherein the input data (I) comprise MRI images (MRI⁰, MRI¹), wherein the MRI images (MRI⁰, MRI¹) are restricted to:
∘ at least one first MRI image (MRI¹), wherein the at least one first MRI image (MRI¹) represents a liver or a part of the liver of the examination object at a point in time in the transitional phase after an administration of a hepatobiliary contrast agent, and
∘ optionally at least one native MRI image (MRI⁰) of the liver or the part of the liver of the examination object without contrast agent,
wherein the target data (T) comprise a second MRI image (MRI²), wherein the second MRI image (MRI²) represents the liver or the part of the liver of the examination object at a point in time in the hepatobiliary phase after the administration of the hepatobiliary contrast agent,
• receiving patient data (PD), wherein the patient data (PD) comprise at least one MRI image (MRI_{P}⁰, MRI_{P}¹), wherein the at least one MRI image (MRI_{P}⁰, MR1_{P}¹) is restricted to at least one first MRI image (MRI_{P}¹) and optionally at least one native MRI image (MRI_{P}⁰), wherein the at least one first MRI image (MRI_{P}¹) represents a liver or a part of the liver of a patient at a point in time in the transitional phase after an administration of a hepatobiliary contrast agent, wherein the optional at least one native MRI image (MRI_{P}⁰) represents the liver or the part of the liver of the patient without contrast agent,
• inputting the patient data (PD) into the trained machine learning model (MLM^{t}),
• receiving a predicted MRI image (MRI_{P}^{2*}) from the trained machine learning model (MLM^{t}), wherein the predicted MRI image (MRI_{P}^{2*}) represents the liver or the part of the liver of the patient at a point in time in the hepatobiliary phase after the administration of the hepatobiliary contrast agent,
• outputting and/or storing the predicted MRI image (MRI_{P}^{2*}) and/or transmitting the predicted MRI image (MRI_{P}^{2*}) to a separate computer system.

2. Method according to Claim 1, wherein the training of the machine learning model (MLM^{t}) comprises:
• receiving and/or providing the training data (TD),
• training the machine learning model (MLM), wherein the machine learning model (MLM) is configured to generate a predicted second MRI image (MRI^{2*}) on the basis of at least one first MRI image (MRI¹), optionally at least one native MRI image (MRI⁰), and model parameters (MP), wherein the training comprises, for each examination object of the plurality of examination objects:
∘ inputting the input data (I) into the machine learning model (MLM),
∘ receiving a predicted second MRI image (MRI^{2*}) from the machine learning model (MLM),
∘ calculating a deviation between the second MRI image (MRI²) and the predicted second MRI image (MRI^{2*}),
∘ modifying the model parameters (MP) with regard to reducing the deviation,
• storing and/or outputting the trained machine learning model (MLM^{t}) and/or transmitting the trained machine learning model (MLM^{t}) to a separate computer system and/or using the trained machine learning model (MLM^{t}) for prediction.

3. Method according to Claim 1 or 2, wherein the examination object is a human.

4. Method according to any one of Claims 1 to 3, wherein the at least one first MRI image (MRI_{P}¹, MRI¹) represents the liver or the part of the liver of the patient/examination object 3 to 6 minutes after the administration of the contrast agent.

5. Method according to any one of Claims 1 to 4, wherein the at least one first MRI image (MRI_{P}¹, MRI¹) is at least one T1-weighted MRI image or comprises such an image.

6. Method according to any one of Claims 1 to 5, wherein the at least one first MRI image (MRI_{P}¹, MRI¹) is the result of a Dixon sequence, preferably a T1-weighted Dixon sequence.

7. Method according to any one of Claims 1 to 6, wherein the at least one first MRI image (MRI_{P}¹, MRI¹) comprises an in-phase image and/or an opposed-phase image and/or a fat-only image and/or a water-only image of the liver or the part of the liver.

8. Method according to any one of Claims 1 to 7, wherein the input data (I) and/or patient data (PD) comprise at least one native MRI image (MRI⁰, MRI_{P}⁰), wherein the at least one native MRI image (MRI⁰, MRI_{P}⁰) is the result of a Dixon sequence, preferably a T1-weighted Dixon sequence, preferably using in- and opposed-phase recording technology.

9. Method according to any one of Claims 1 to 8, wherein the second MRI image (MRI_{P}², MRI²) is a representation of the liver at a point in time in the range of 10 minutes to 30 minutes, preferably 15 minutes to 25 minutes, after the administration of the hepatobiliary contrast agent.

10. Method according to any one of Claims 1 to 9, wherein the second MRI image (MRI_{P}², MRI²) is a T1-weighted MRI image, preferably a water-only image.

11. Method according to any one of Claims 1 to 10, wherein the contrast agent comprises:
- the disodium salt of gadoxetic acid,
- gadolinium 2,2',2"-(10-{1-carboxy-2-[2-(4-ethoxyphenyl)ethoxy]ethyl}-1,4,7,10-tetraazacyclododecane-1,4,7-triyl)triacetate,
- gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate,
- gadolinium 2,2',2"-{10-[(1R)-1-carboxy-2-{4-[2-(2-ethoxyethoxy)ethoxy]phenyl}ethyl]-1,4,7,10-
- gadolinium (2S,2'S,2''S)-2,2',2''-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate), or
- gadolinium 2,2',2"-{10-[(1S)-4-(4-butoxyphenyl)-1-carboxybutyl]-1,4,7,10-tetraazacyclododecane-1,4,7-triylltriacetate.

12. Computer system (10), comprising
• an input unit (11),
• a control and calculation unit (12), and
• an output unit (13),
wherein the control and calculation unit (12) is configured
• to cause the input unit (11) to receive patient data (PD), wherein the patient data (PD) comprise at least one MRI image (MRI_{P}⁰, MRI_{P}¹), wherein the at least one MRI image (MRI_{P}⁰, MR1_{P}¹) is restricted to at least one first MRI image (MRI_{P}¹) and optionally at least one native MRI image (MRI_{P}⁰), wherein the at least one first MRI image (MRI_{P}¹) represents a liver or a part of the liver of a patient at a point in time in the transitional phase after an administration of a hepatobiliary contrast agent, wherein the optional at least one native MRI image (MRI_{P}⁰) represents the liver or the part of the liver of the patient without contrast agent,
• to input the patient data (PD) into a trained machine learning model (MLM^{t}), wherein the trained machine learning model (MLM^{t}) was trained on the basis of training data (TD) to generate a predicted second MRI image (MRI^{2*}) on the basis of at least one first MRI image (MRI¹) and optionally at least one native MRI image (MRI⁰), wherein the training data (TD) comprise input data (I) and target data (T) for each examination object of a plurality of examination objects, wherein the input data (I) comprise MRI images (MRI⁰, MRI¹),
wherein the MRI images (MRI⁰, MRI¹) are restricted to:
∘ at least one first MRI image (MRI¹), wherein the at least one first MRI image (MRI¹) represents a liver or a part of the liver of the examination object at a point in time in the transitional phase after an administration of a hepatobiliary contrast agent, and
∘ optionally at least one native MRI image (MRI⁰) of the liver or the part of the liver of the examination object without contrast agent,
wherein the target data (T) comprise a second MRI image (MRI²), wherein the second MRI image (MRI²) represents the liver or the part of the liver of the examination object at a point in time in the hepatobiliary phase after the administration of the hepatobiliary contrast agent,
• to receive a predicted MRI image (MRI_{P}^{2*}) from the trained machine learning model (MLM^{t}), wherein the predicted MRI image (MRI_{P}^{2*}) represents the liver or the part of the liver of the patient at a point in time in the hepatobiliary phase after the administration of the hepatobiliary contrast agent,
• to cause the output unit (13) to output the predicted MRI image (MRI_{P}^{2*}) and/or to store it and/or to transmit it to a separate computer system.

13. Computer program product comprising a data memory in which a computer program (60) is stored that can be loaded into a working memory (50) of a computer system (10), where it causes the computer system (10) to execute the following steps:
• receiving patient data (PD), wherein the patient data (PD) comprise at least one MRI image (MRI_{P}⁰, MRI_{P}¹), wherein the at least one MRI image (MRI_{P}⁰, MRI_{P}¹) is restricted to at least one first MRI image (MRI_{P}¹) and optionally at least one native MRI image (MRI_{P}⁰), wherein the at least one first MRI image (MRI_{P}¹) represents a liver or a part of the liver of a patient at a point in time in the transitional phase after an administration of a hepatobiliary contrast agent, wherein the optional at least one native MRI image (MRI_{P}⁰) represents the liver or the part of the liver of the patient without contrast agent,
• inputting the patient data (PD) into a trained machine learning model (MLM^{t}), wherein the machine learning model (MLM^{t}) was trained on the basis of training data (TD) to generate a predicted second MRI image (MRI^{2*}) on the basis of at least one first MRI image (MRI¹) and optionally at least one native MRI image (MRI⁰), wherein the training data (TD) comprise input data (I) and target data (T) for each examination object of a plurality of examination objects, wherein the input data (I) comprise MRI images (MRI⁰, MRI¹),
wherein the MRI images (MRI⁰, MRI¹) are restricted to:
∘ at least one first MRI image (MRI¹), wherein the at least one first MRI image (MRI¹) represents a liver or a part of the liver of the examination object at a point in time in the transitional phase after an administration of a hepatobiliary contrast agent, and
∘ optionally at least one native MRI image (MRI⁰) of the liver or the part of the liver of the examination object without contrast agent,
wherein the target data (T) comprise a second MRI image (MRI²), wherein the second MRI image (MRI²) represents the liver or the part of the liver of the examination object at a point in time in the hepatobiliary phase after the administration of the hepatobiliary contrast agent,
• receiving a predicted MRI image (MRI_{P}^{2*}) from the trained machine learning model (MLM^{t}), wherein the predicted MRI image (MRI_{P}^{2*}) represents the liver or the part of the liver of the patient at a point in time in the hepatobiliary phase after the administration of the hepatobiliary contrast agent,
• outputting and/or storing the predicted MRI image (MRI_{P}^{2*}) and/or transmitting the predicted MRI image (MRI_{P}^{2*}) to a separate computer system.

14. Use of a hepatobiliary contrast agent in an MRI examination method, comprising:
• receiving and/or generating patient data (PD), wherein the patient data (PD) comprise at least one MRI image (MRI_{P}⁰, MRI_{P}¹), wherein the at least one MRI image (MRI_{P}⁰, MRI_{P}¹) is restricted to at least one first MRI image (MRI_{P}¹) and optionally at least one native MRI image (MRI_{P}⁰), wherein the at least one first MRI image (MRI_{P}¹) represents a liver or a part of the liver of a patient at a point in time in the transitional phase after an administration of the hepatobiliary contrast agent, wherein the optional at least one native MRI image (MRI_{P}⁰) represents the liver or the part of the liver of the patient without contrast agent,
• inputting the patient data (PD) into a trained machine learning model (MLM^{t}), wherein the machine learning model (MLM^{t}) was trained on the basis of training data (TD) to generate a predicted second MRI image (MRI²*) on the basis of at least one first MRI image (MRI¹) and optionally at least one native MRI image (MRI⁰), wherein the training data (TD) comprise input data (I) and target data (T) for each examination object of a plurality of examination objects, wherein the input data (I) comprise MRI images (MRI⁰, MRI¹),
wherein the MRI images (MRI⁰, MRI¹) are restricted to:
∘ at least one first MRI image (MRI¹), wherein the at least one first MRI image (MRI¹) represents a liver or a part of the liver of the examination object at a point in time in the transitional phase after an administration of a hepatobiliary contrast agent, and
∘ optionally at least one native MRI image (MRI⁰) of the liver or the part of the liver of the examination object without contrast agent,
wherein the target data (T) comprise a second MRI image (MRI²), wherein the second MRI image (MRI²) represents the liver or the part of the liver of the examination object at a point in time in the hepatobiliary phase after the administration of the hepatobiliary contrast agent,
• receiving a predicted MRI image (MRI_{P}^{2*}) from the trained machine learning model (MLM^{t}), wherein the predicted MRI image (MRI_{P}²*) represents the liver or the part of the liver of the patient at a point in time in the hepatobiliary phase after the administration of the hepatobiliary contrast agent,
• outputting and/or storing the predicted MRI image (MRI_{P}^{2*}) and/or transmitting the predicted MRI image (MRI_{P}^{2*}) to a separate computer system.

15. Kit comprising a hepatobiliary contrast agent and a computer program product, wherein the computer program product comprises a data memory in which a computer program (60) is stored that can be loaded into a working memory (50) of a computer system (10), where it causes the computer system (10) to execute the following steps:
• receiving patient data (PD), wherein the patient data (PD) comprise at least one MRI image (MRI_{P}⁰, MRI_{P}¹), wherein the at least one MRI image (MRI_{P}⁰, MR1_{P}¹) is restricted to at least one first MRI image (MRI_{P}¹) and optionally at least one native MRI image (MRI_{P}⁰), wherein the at least one first MRI image (MRI_{P}¹) represents a liver or a part of the liver of a patient at a point in time in the transitional phase after an administration of the hepatobiliary contrast agent, wherein the optional at least one native MRI image (MRI_{P}⁰) represents the liver or the part of the liver of the patient without contrast agent,
• inputting the patient data (PD) into a trained machine learning model (MLM^{t}), wherein the machine learning model (MLM^{t}) was trained on the basis of training data (TD) to generate a predicted second MRI image (MRI²*) on the basis of at least one first MRI image (MRI¹) and optionally at least one native MRI image (MRI⁰), wherein the training data (TD) comprise input data (I) and target data (T) for each examination object of a plurality of examination objects, wherein the input data (I) comprise MRI images (MRI⁰, MRI¹),
wherein the MRI images (MRI⁰, MRI¹) are restricted to:
∘ at least one first MRI image (MRI¹), wherein the at least one first MRI image (MRI¹) represents a liver or a part of the liver of the examination object at a point in time in the transitional phase after an administration of a hepatobiliary contrast agent, and
∘ optionally at least one native MRI image (MRI⁰) of the liver or the part of the liver of the examination object without contrast agent,
wherein the target data (T) comprise a second MRI image (MRI²), wherein the second MRI image (MRI²) represents the liver or the part of the liver of the examination object at a point in time in the hepatobiliary phase after the administration of the hepatobiliary contrast agent,
• receiving a predicted MRI image (MRI_{P}^{2*}) from the trained machine learning model (MLM^{t}), wherein the predicted MRI image (MRI_{P}^{2*}) represents the liver or the part of the liver of the patient at a point in time in the hepatobiliary phase after the administration of the hepatobiliary contrast agent,
• outputting and/or storing the predicted MRI image (MRI_{P}^{2*}) and/or transmitting the predicted MRI image (MRI_{P}^{2*}) to a separate computer system.

## Revendications

1. Procédé mis en œuvre par ordinateur, comprenant les étapes consistant à :
• fournir un modèle entraîné (MLM^{t}) d'apprentissage automatique, le modèle entraîné (MLM^{t}) d'apprentissage automatique ayant été entraîné à l'aide de données d'apprentissage (TD), sur la base d'au moins une première image IRM (MRI¹) et facultativement d'au moins une image IRM native (MRI⁰) pour générer une seconde image IRM prédite (MRI^{2*}), les données d'apprentissage (TD), pour chaque objet d'examen d'une pluralité d'objets d'examen, comprenant des données d'entrée (I) et des données cibles (T),
les données d'entrée (I) comprenant des images IRM (MRI⁰, MRI¹), les images IRM (MRI⁰, MRI¹) étant limitées à :
∘ au moins une première image IRM (MRI¹), ladite au moins une première image IRM (MRI¹) représentant un foie ou une partie du foie de l'objet d'examen à un instant donné dans la phase de transition après une administration d'un agent de contraste hépatobiliaire, et
∘ facultativement, au moins une image IRM (MRI⁰) du foie ou de la partie du foie de l'objet d'examen sans agent de contraste,
les données cibles (T) comprenant une seconde image IRM (MRI²), la seconde image IRM (MRI²) représentant le foie ou la partie du foie de l'objet d'examen à un instant donné dans la phase hépatobiliaire après l'administration de l'agent de contraste hépatobiliaire,
• recevoir des données de patient (PD), les données de patient (PD) comprenant au moins une image IRM (MRI_{P}⁰, MRI_{P}¹), ladite au moins une image IRM (MRI_{P}⁰, MRI_{P}¹) étant limitée à au moins une première image IRM (MRI_{P}¹) et facultativement à au moins une image IRM native (MRI_{P}⁰), ladite au moins une première image IRM (MRI_{P}¹) représentant un foie ou une partie du foie d'un patient à un instant donné dans la phase de transition après une administration d'un agent de contraste hépatobiliaire, ladite au moins une image IRM native facultative (MRI_{P}⁰) représentant le foie ou la partie du foie du patient sans agent de contraste,
• introduire les données de patient (PD) dans le modèle (MLM^{t}) d'apprentissage automatique,
• recevoir une image IRM prédite (MRI_{P}^{2*}) en provenance du modèle entraîné (MLM^{t}) d'apprentissage automatique, l'image IRM prédite (MRI_{P}^{2*}) représentant le foie ou la partie du foie du patient à un instant donné dans la phase hépatobiliaire après l'administration de l'agent de contraste hépatobiliaire,
• fournir en sortie et/ou stocker l'image IRM prédite (MRI_{P}^{2*}) et/ou transmettre l'image IRM prédite (MRI_{P}^{2*}) à un système informatique séparé.

2. Procédé selon la revendication 1, dans lequel l'entraînement du modèle (MLM^{t}) d'apprentissage automatique comprend les étapes consistant à :
• recevoir et/ou fournir les données d'apprentissage (TD),
• entraîner le modèle (MLM) d'apprentissage automatique, le modèle (MLM) d'apprentissage automatique étant configuré pour générer une seconde image IRM prédite (MRI^{2*}) sur la base d'au moins une première image IRM (MRI¹), facultativement d'au moins une image IRM native (MRI⁰) et de paramètres de modèle (MP), l'apprentissage, pour chaque objet d'examen de la pluralité d'objets d'examen, comprenant les étapes consistant à :
∘ introduire les données d'entrée (I) dans le modèle (MLM) d'apprentissage automatique,
∘ recevoir une seconde image IRM prédite (MRI^{2*}) en provenance du modèle (MLM) d'apprentissage automatique,
∘ calculer un écart entre la seconde image IRM (MRI²) et la seconde image IRM prédite (MRI^{2*}),
∘ modifier les paramètres de modèle (MP) en vue de réduire l'écart,
• stocker et/ou fournir en sortie le modèle entraîné (MLM^{t}) d'apprentissage automatique et/ou transmettre le modèle entraîné (MLM^{t}) d'apprentissage automatique à un système informatique séparé et/ou utiliser le modèle entraîné (MLM^{t}) d'apprentissage automatique à des fins de prédiction.

3. Procédé selon la revendication 1 ou 2, dans lequel l'objet d'examen est un être humain.

4. Procédé selon l'une quelconque des revendications 1 à 3, dans lequel ladite au moins une première image IRM (MRI_{P}¹, MRI¹) représente le foie ou la partie du foie du patient/de l'objet d'examen 3 à 6 minutes après l'administration de l'agent de contraste.

5. Procédé selon l'une quelconque des revendications 1 à 4, dans lequel ladite au moins une première image IRM (MRI_{P}¹, MRI¹) est ou comprend au moins une image IRM pondérée en T1.

6. Procédé selon l'une quelconque des revendications 1 à 5, dans lequel ladite au moins une première image IRM (MRI_{P}¹, MRI¹) est le résultat d'une séquence Dixon, de préférence d'une séquence Dixon pondérée en T1.

7. Procédé selon l'une quelconque des revendications 1 à 6, dans lequel ladite au moins une première image IRM (MRI_{P}¹, MRI¹) comprend une image en phase et/ou une image en opposition de phase et/ou une carte des graisses et/ou une carte de l'eau du foie ou de la partie du foie.

8. Procédé selon l'une quelconque des revendications 1 à 7, dans lequel les données d'entrée (I) et/ou les données de patient (PD) comprennent au moins une image IRM native (MRI⁰, MRI_{P}⁰), ladite au moins une image IRM native (MRI⁰, MRI_{P}⁰) étant le résultat d'une séquence Dixon, de préférence une séquence Dixon pondérée en T1, de préférence par une technique d'acquisition en phase et en opposition de phase.

9. Procédé selon l'une quelconque des revendications 1 à 8, dans lequel la seconde image IRM (MRI_{P}², MRI²) est une représentation du foie à un instant se situant dans la plage de 10 minutes à 30 minutes, de préférence de 15 minutes à 25 minutes, après l'administration de l'agent de contraste hépatobiliaire.

10. Procédé selon l'une quelconque des revendications 1 à 9, dans lequel la seconde image IRM (MRI_{P}², MRI²) est une image IRM pondérée en T1, de préférence une carte de l'eau.

11. Procédé selon l'une quelconque des revendications 1 à 10, dans lequel l'agent de contraste comprend :
- le sel disodique de l'acide gadoxétique,
- 2,2',2"-(10-{1-carboxy-2-[2-(4-éthoxyphényl)éthoxy]éthyl}-1,4,7,10-tétraazacyclododécan-1,4,7-triyl)triacétate de gadolinium,
- 2,2',2"-{10-[1-carboxy-2-{4-[2-(2-éthoxyéthoxy)éthoxy]phényl}éthyl]-1,4,7,10-tétraazacyclododécan-1,4,7-triyl}triacétate de gadolinium,
- 2,2',2"-{10-[(1R)-1-carboxy-2-{4-[2-(2-éthoxyéthoxy)éthoxy]phényl}éthyl]-1,4,7,10- de gadolinium
- (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécan-1,4,7-triyl}tris(3-hydroxypropanoate) de gadolinium ou
- 2,2',2"-{10-[(1S)-4-(4-butoxyphényl)-1-carboxybutyl]-1,4,7,10-tétraazacyclododécan-1,4,7-triyl}triacétate de gadolinium.

12. Système informatique (10) comprenant
• une unité d'entrée (11),
• une unité de commande et de calcul (12), et
• une unité de sortie (13),
l'unité de commande et de calcul (12) étant configurée pour
• amener l'unité d'entrée (11) à recevoir des données de patient (PD), les données de patient (PD) comprenant au moins une image IRM (MRI_{P}⁰, MRI_{P}¹), ladite au moins une image IRM (MR1_{P}⁰, MRI_{P}¹) étant limitée à au moins une première image IRM (MRI_{P}¹) et facultativement à au moins une image IRM native (MRI_{P}⁰), ladite au moins une première image IRM (MRI_{P}¹) représentant un foie ou une partie du foie d'un patient à un instant donné dans la phase de transition après une administration d'un agent de contraste hépatobiliaire, ladite au moins une image IRM native facultative (MRI_{P}⁰) représentant le foie ou la partie du foie du patient sans agent de contraste,
• introduire les données de patient (PD) dans un modèle (MLM^{t}) d'apprentissage automatique, le modèle (MLM^{t}) d'apprentissage automatique ayant été entraîné à l'aide de données d'apprentissage (TD), sur la base d'au moins une première image IRM (MRI¹) et facultativement d'au moins une image IRM native (MRI⁰) pour générer une seconde image IRM prédite (MRI^{2*}), les données d'apprentissage (TD), pour chaque objet d'examen d'une pluralité d'objets d'examen, comprenant des données d'entrée (I) et des données cibles (T), les données d'entrée (I) comprenant des images IRM (MRI⁰, MRI¹),
les images IRM (MRI⁰, MRI¹) étant limitées à :
∘ au moins une première image IRM (MRI¹), ladite au moins une première image IRM (MRI¹) représentant un foie ou une partie du foie de l'objet d'examen à un instant donné dans la phase de transition après une administration d'un agent de contraste hépatobiliaire, et
∘ facultativement, au moins une image IRM (MRI⁰) du foie ou de la partie du foie de l'objet d'examen sans agent de contraste,
les données cibles (T) comprenant une seconde image IRM (MRI²), la seconde image IRM (MRI²) représentant le foie ou la partie du foie de l'objet d'examen à un instant donné dans la phase hépatobiliaire après l'administration de l'agent de contraste hépatobiliaire,
• recevoir une image IRM prédite (MRI_{P}^{2*}) en provenance du modèle entraîné (MLM^{t}) d'apprentissage automatique, l'image IRM prédite (MRI_{P}^{2*}) représentant le foie ou la partie du foie du patient à un instant donné dans la phase hépatobiliaire après l'administration de l'agent de contraste hépatobiliaire,
• amener l'unité de sortie (13) à fournir en sortie et/ou à stocker et/ou à transmettre l'image IRM prédite (MRI_{P}^{2*}) à un système informatique séparé.

13. Produit de programme informatique, comprenant un support de données sur lequel est stocké un programme informatique (60), qui peut être chargé dans une mémoire vive (50) d'un système informatique (10) et y amenant le système informatique (10) à exécuter les étapes suivantes consistant à :
• recevoir des données de patient (PD), les données de patient (PD) comprenant au moins une image IRM (MRI_{P}⁰, MRI_{P}¹), ladite au moins une image IRM (MRI_{P}⁰, MRI_{P}¹) étant limitée à au moins une première image IRM (MRI_{P}¹) et facultativement à au moins une image IRM native (MRI_{P}⁰), ladite au moins une première image IRM (MRI_{P}¹) représentant un foie ou une partie du foie d'un patient à un instant donné dans la phase de transition après une administration d'un agent de contraste hépatobiliaire, ladite au moins une image IRM native facultative (MRI_{P}⁰) représentant le foie ou la partie du foie du patient sans agent de contraste,
• introduire les données de patient (PD) dans un modèle (MLM^{t}) d'apprentissage automatique, le modèle (MLM^{t}) d'apprentissage automatique ayant été entraîné à l'aide de données d'apprentissage automatique (TD), sur la base d'au moins une première image IRM (MRI¹) et facultativement d'au moins une image IRM native (MRI⁰) pour générer une seconde image IRM prédite (MRI^{2*}), les données d'apprentissage (TD), pour chaque objet d'examen d'une pluralité d'objets d'examen, comprenant des données d'entrée (I) et des données cibles (T), les données d'entrée (I) comprenant des images IRM (MRI⁰, MRI¹),
les images IRM (MRI⁰, MRI¹) étant limitées à :
∘ au moins une première image IRM (MRI¹), ladite au moins une première image IRM (MRI¹) représentant un foie ou une partie du foie de l'objet d'examen à un instant donné dans la phase de transition après une administration d'un agent de contraste hépatobiliaire, et
∘ facultativement, au moins une image IRM (MRI⁰) du foie ou de la partie du foie de l'objet d'examen sans agent de contraste,
les données cibles (T) comprenant une seconde image IRM (MRI²), la seconde image IRM (MRI²) représentant le foie ou la partie du foie de l'objet d'examen à un instant donné dans la phase hépatobiliaire après l'administration de l'agent de contraste hépatobiliaire,
• recevoir une image IRM prédite (MRI_{P}^{2*}) en provenance du modèle entraîné (MLM^{t}) d'apprentissage automatique, l'image IRM prédite (MRI_{P}^{2*}) représentant le foie ou la partie du foie du patient à un instant donné dans la phase hépatobiliaire après l'administration de l'agent de contraste hépatobiliaire,
• fournir en sortie et/ou stocker l'image IRM prédite (MRI_{P}^{2*}) et/ou transmettre l'image IRM prédite (MRI_{P}^{2*}) à un système informatique séparé.

14. Utilisation d'un agent de contraste hépatobiliaire dans un procédé d'examen IRM comprenant les étapes consistant à :
• recevoir et/ou générer des données de patient (PD), les données de patient (PD) comprennent au moins une image IRM (MRI_{P}⁰, MRI_{P}¹), ladite au moins une image IRM (MRI_{P}⁰, MRI_{P}¹) étant limitée à au moins une première image IRM (MRI_{P}¹) et facultativement à au moins une image IRM native (MRI_{P}⁰), ladite au moins une première image IRM (MRI_{P}¹) représentant un foie ou une partie du foie d'un patient à un instant donné dans la phase de transition après une administration de l'agent de contraste hépatobiliaire, ladite au moins une image IRM native facultative (MRI_{P}⁰) représentant le foie ou la partie du foie du patient sans agent de contraste,
• introduire les données de patient (PD) dans un modèle (MLM^{t}) d'apprentissage automatique, le modèle (MLM^{t}) d'apprentissage automatique ayant été entraîné à l'aide de données d'apprentissage automatique (TD), sur la base d'au moins une première image IRM (MRI¹) et facultativement d'au moins une image IRM native (MRI⁰) pour générer une seconde image IRM prédite (MRI^{2*}), les données d'apprentissage (TD), pour chaque objet d'examen d'une pluralité d'objets d'examen, comprenant des données d'entrée (I) et des données cibles (T), les données d'entrée (I) comprenant des images IRM (MRI⁰, MRI¹),
les images IRM (MRI⁰, MRI¹) étant limitées à :
∘ au moins une première image IRM (MRI¹), ladite au moins une première image IRM (MRI¹) représentant un foie ou une partie du foie de l'objet d'examen à un instant donné dans la phase de transition après une administration d'un agent de contraste hépatobiliaire, et
∘ facultativement, au moins une image IRM (MRI⁰) du foie ou de la partie du foie de l'objet d'examen sans agent de contraste,
les données cibles (T) comprenant une seconde image IRM (MRI²), la seconde image IRM (MRI²) représentant le foie ou la partie du foie de l'objet d'examen à un instant donné dans la phase hépatobiliaire après l'administration de l'agent de contraste hépatobiliaire,
• recevoir une image IRM prédite (MRI_{P}^{2*}) en provenance du modèle entraîné (MLM^{t}) d'apprentissage automatique, l'image IRM prédite (MRI_{P}^{2*}) représentant le foie ou la partie du foie du patient à un instant donné dans la phase hépatobiliaire après l'administration de l'agent de contraste hépatobiliaire,
• fournir en sortie et/ou stocker l'image IRM prédite (MRI_{P}^{2*}) et/ou transmettre l'image IRM prédite (MRI_{P}^{2*}) à un système informatique séparé.

15. Kit comprenant un agent de contraste et un produit de programme informatique, le produit de programme informatique comprenant une mémoire de données dans laquelle est stocké un programme informatique (60) qui peut être chargé dans une mémoire de travail (50) d'un système informatique (10) et qui y amène le système informatique (10) à exécuter les étapes suivantes consistant à :
• recevoir des données de patient (PD), les données de patient (PD) comprenant au moins une image IRM (MRI_{P}⁰, MRI_{P}¹), ladite au moins une image IRM (MRI_{P}⁰, MRI_{P}¹) étant limitée à au moins une première image IRM (MRI_{P}¹) et facultativement à au moins une image IRM native (MRI_{P}⁰), ladite au moins une première image IRM (MRI_{P}¹) représentant un foie ou une partie du foie d'un patient à un instant donné dans la phase de transition après une administration de l'agent de contraste hépatobiliaire, ladite au moins une image IRM native facultative (MRI_{P}⁰) représentant le foie ou la partie du foie du patient sans agent de contraste,
• introduire les données de patient (PD) dans un modèle (MLM^{t}) d'apprentissage automatique, le modèle (MLM^{t}) d'apprentissage automatique ayant été entraîné à l'aide de données d'apprentissage automatique (TD), sur la base d'au moins une première image IRM (MRI¹) et facultativement d'au moins une image IRM native (MRI⁰) pour générer une seconde image IRM prédite (MRI^{2*}), les données d'apprentissage (TD), pour chaque objet d'examen d'une pluralité d'objets d'examen, comprenant des données d'entrée (I) et des données cibles (T), les données d'entrée (I) comprenant des images IRM (MRI⁰, MRI¹),
les images IRM (MRI⁰, MRI¹) étant limitées à :
∘ au moins une première image IRM (MRI¹), ladite au moins une première image IRM (MRI¹) représentant un foie ou une partie du foie de l'objet d'examen à un instant donné dans la phase de transition après une administration d'un agent de contraste hépatobiliaire, et
∘ facultativement, au moins une image IRM (MRI⁰) du foie ou de la partie du foie de l'objet d'examen sans agent de contraste,
les données cibles (T) comprenant une seconde image IRM (MRI²), la seconde image IRM (MRI²) représentant le foie ou la partie du foie de l'objet d'examen à un instant donné dans la phase hépatobiliaire après l'administration de l'agent de contraste hépatobiliaire,
• recevoir une image IRM prédite (MRI_{P}^{2*}) en provenance du modèle entraîné (MLM^{t}) d'apprentissage automatique, l'image IRM prédite (MRI_{P}^{2*}) représentant le foie ou la partie du foie du patient à un instant donné dans la phase hépatobiliaire après l'administration de l'agent de contraste hépatobiliaire,
• fournir en sortie et/ou stocker l'image IRM prédite (MRI_{P}^{2*}) et/ou transmettre l'image IRM prédite (MRI_{P}^{2*}) à un système informatique séparé.
